(19) Europäisches Patentamt
European Patent Office
Office européen des brevets

(11) **EP 4 506 037 A1**

(12) **EUROPEAN PATENT APPLICATION**
published in accordance with Art. 153(4) EPC

(43) Date of publication:
**12.02.2025 Bulletin 2025/07**

(21) Application number: **23784681.1**

(22) Date of filing: **28.03.2023**

(51) International Patent Classification (IPC):
*A61Q 1/06* (2006.01)        *A61Q 1/10* (2006.01)
*A61Q 1/12* (2006.01)        *A61Q 17/04* (2006.01)
*A61Q 19/00* (2006.01)        *A61K 8/23* (2006.01)
*A61K 8/25* (2006.01)        *A61K 8/36* (2006.01)

(52) Cooperative Patent Classification (CPC):
A61K 8/23; A61K 8/25; A61K 8/36; A61Q 1/06;
A61Q 1/10; A61Q 1/12; A61Q 17/04; A61Q 19/00

(86) International application number:
**PCT/JP2023/012586**

(87) International publication number:
**WO 2023/195391 (12.10.2023 Gazette 2023/41)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.04.2022 JP 2022063574**

(71) Applicant: **Iwase Cosfa Co., Ltd.
Osaka-shi, Osaka 541-0045 (JP)**

(72) Inventors:
• **TANAKA, Ippei
Osaka-shi, Osaka 541-0045 (JP)**
• **KATAYAMA, Nohara
Osaka-shi, Osaka 541-0045 (JP)**

(74) Representative: **Müller-Boré & Partner
Patentanwälte PartG mbB
Friedenheimer Brücke 21
80639 München (DE)**

(54) **COSMETIC**

(57) A novel cosmetic that exhibits an excellent extension when applied, also an excellent adherence post-application and/or an excellent finish post-application, as well as a good use sensation, can be provided by a composite particle-containing cosmetic wherein: the composite particle contains an inorganic nucleating agent and a metal soap; the inorganic nucleating agent is at least one selected from the group consisting of glass particles and sparingly soluble sulfate salt particles; and the metal soap is the salt of a fatty acid and at least one selected from the group consisting of calcium and magnesium.

EP 4 506 037 A1

**Description**

Technical Field

The present disclosure relates to a cosmetic.

Background Art

Patent Literature 1 discloses a solid powder cosmetic using a metal soap that can provide high dispersibility and covering property to powder and can enhance anti-caking property of powder, flowability and feeling.

Citation List

Patent Literature

Patent Literature 1: WO2016/132967

Summary of Invention

Technical Problem

Though Patent Literature 1 discloses that metal soap particles consisting of particular inorganic particles and metal soap exhibit good spreading property, there is no description nor suggestion about adherence to skin, lips, eyelashes and the like after application and about finish that are important properties in cosmetics. Further, in Patent literature 1, there is no description nor suggestion about formability, rubbing-off property, anti-caking etc. that are important properties in solid powder cosmetics as represented by for example powder foundation.
The objective of the present disclosure is to provide a novel cosmetic that exhibits an excellent spreading when applied, also excellent adherence after application and excellent finish after application, as well as good feeling of use.

Solution to Problem

The present disclosure includes the following aspects:

[Item 1] A cosmetic comprising composite particles, wherein

the composite particles contain an inorganic nucleating agent a metal soap;
the inorganic nucleating agent is at least one selected from the group consisting of glass particles and sparingly soluble sulfate salt particles;
the metal soap is a salt of a fatty acid and at least one selected from the group consisting of calcium and magnesium.

[Item 2] The cosmetic according to item 1, wherein an amount of the inorganic nucleating agent in the composite particles is 0.1 wt% or more and 30 wt% or less.
[Item 3] The cosmetic according to item 1 or 2, wherein an inside incorporation rate A represented by the following Formula (1) is 25% or more:

$$\text{Inside incorporation rate } A = 100 - (X/X') \times 100$$

$$\text{Formula (1)}$$

wherein,

X is a surface content of the inorganic nucleating agent in the composite particles,
X' is a whole content of the inorganic nucleating agent in the composite particles,
X and X' are average values obtained by conducting elemental analysis using SEM/EDX on 3 places within an area of 15 $\mu$m square under the condition of an acceleration voltage of 10 kV and a height of 15 mm for the composite particles before and after homogenization respectively.

[Item 4] The cosmetic according to any one of items 1 to 3, wherein a grain size summary value B represented by the following Formula (2) is 3 or less:

$$\text{grain size summary value B} = (D90 - D10)/D50$$

$$\text{Formula (2)}$$

wherein,

D10 is a 10% cumulative diameter ($\mu$m) of the composite particles on the basis of volume,
D50 is a 50% cumulative diameter ($\mu$m) of the composite particles on the basis of volume, and
D90 is a 90% cumulative diameter ($\mu$m) of the composite particles on the basis of volume.

[Item 5] The cosmetic according to any one of items 1 to 4, wherein the glass particles are silicate glass particles.
[Item 6] The cosmetic according to any one of items 1 to 5, wherein the inorganic nucleating agent is at least one selected from the group consisting of borosilicate glass particles and barium sulfate particles.
[Item 7] The cosmetic according to any one of items 1 to 6, wherein the number of carbon atoms of the fatty acid is 8 or more and 22 or less.
[Item 8] The solid powder cosmetic according to any one of items 1 to 7, wherein an amount of the composite particles in the cosmetic is 1 wt% or more and 75 wt% or less.
[Item 9] The cosmetic according to any one of items 1 to 8, wherein

the cosmetic further contains an oily component, and
an amount of the oily component in the cosmetic is 0.1 wt% or more and 20 wt% or less.

[Item 10] The cosmetic according to any one of items 1 to 9, wherein

the cosmetic further contains spherical particles,
50% cumulative value of the spherical particles on the basis of volume is 1 $\mu$m or more and 30 $\mu$m or less, and
an amount of the spherical particles in the cosmetic is 1 wt% or more.

[Item 11] The cosmetic according to any one of items 1 to 10, wherein the cosmetic is a solid powder cosmetic.

Advantageous Effect of Invention

According to the present disclosure, a novel cosmetic can be provided that exhibits an excellent spreading when applied, also excellent adherence after application and excellent finish after application, as well as good feeling of use.

Description of Embodiments

<Cosmetic>

The cosmetic of the present disclosure contains at least composite particles.

{Composite particles}

The composite particles of the present disclosure contain an inorganic nucleating agent and a metal soap.

[Inorganic nucleating agent]

The inorganic nucleating agent is inorganic particles that can function as nuclei in generating composite particles, and the inorganic nucleating agent may be at least one selected from the group consisting of glass particles and sparingly soluble sulfate salt particles.
The glass particles may be a variety of glass particles having predominantly the inorganic oxide structure of amorphous three-dimensional network shape. For example, the glass particles are preferably those containing one or more of oxides of the elements selected from the group consisting of Si, B, Al, Ba, Ca, Mg, Li, Na, K, Zn, Sr, Sn, Zr, Sb, Bi, Te, Pb, W, Ti, Y, and Ag as constituents. Examples of the glass particles include the glass particles that contain at least one selected from the group consisting of $SiO_2$, $B_2O_3$, $Al_2O_3$, BaO, CaO, MgO, ZnO, SrO, $ZrO_2$, $Bi_2O_3$, PbO, $Y_2O_3$, $TeO_2$, $WO_3$, $TiO_2$, $R_2O$ (R represents an alkaline metal such as Na, K and Li) as suitable examples of constituents. As examples of the glass particles which can be used, silicate glass containing $SiO_2$ and metal components as main components is preferable. In particular, borosilicate glass containing components such as boron oxide ($B_2O_3$) is preferable. Specific examples of the glass particles include silicate glass, borosilicate glass, soda lime glass, soda glass, fused quartz glass, alkali-free glass, aluminosilicate glass, boroaluminosilicate glass, aluminosilicate glass, lithium alumina silicate glass, alkali barium glass, lead glass, bismuth-based glass, and the like. These may be used singly or in combinations of two or more thereof. In one embodiment, borosilicate (Ca/Al) may be used as the glass particles.

The sparingly soluble sulfate salt is a sulfate salt which is sparingly soluble in water. The solubility in water (20 °C) of the sparingly soluble sulfate salt may be 1g/100mL or less, 0.5g/100mL or less, 0.1g/100mL or less, 0.05g/100mL or less, 0.01g/100mL or less, preferably 0.1g/100mL or less. Specific examples of the sparingly soluble sulfate salt include anhydrides or hydrates of barium sulfate, calcium sulfate, lead sulfate, silver sulfate, strontium sulfate and the like. These may be used singly or in combinations of two or more thereof. In one embodiment, barium sulfate may be used as the sparingly soluble sulfate salt.

The 50% cumulative diameter (D50) of the inorganic nucleating agent on the basis of the volume may be 0.1 $\mu$m or more, 1 $\mu$m or more, 3 $\mu$m or more, 5 $\mu$m or more, 10 $\mu$m or more, 20 $\mu$m or more, or 30 $\mu$m or more, preferably 1 $\mu$m or more, more preferably 3 $\mu$m or more, further preferably 5 $\mu$m or more. The 50% cumulative diameter (D50) of the inorganic nucleating agent on the basis of the volume may be 100 $\mu$m or less, 75 $\mu$m or less, 50 $\mu$m or less, 20 $\mu$m or less, 10 $\mu$m or less, or 5 $\mu$m or less, preferably 50 $\mu$m or less, more preferably 20 $\mu$m or less. When the 50% cumulative diameter (D50) is within the range described above, the inside incorporation rate A described below can be enhanced, the grain size of the resulting composite particles can be refined, and the shape of the particles can be made uniform, while the inorganic nucleating agent has the function as crystal nuclei. The particle size distribution of the inorganic nucleating agent may be unimodal or multimodal, for example it is unimodal.

In the present disclosure, a particle size such as 50% cumulative diameter (D50) can be measured by a laser diffraction method. A laser diffraction method is a method for obtaining a particle size using scattered light obtained by irradiating particles with a laser beam. In the present disclosure, measurements can be conducted by a wet process in which a sample is added to a circulating dispersion media, for example an organic solvent such as ethanol and isopropanol. As a measurement apparatus, for example Microtrac MT-3000 manufactured by NIKISO CO., LTD. can be used.

(Amount of Inorganic nucleating agent)

The amount of the inorganic nucleating agent in the composite particles may be 0.1 wt% or more, 0.5 wt% or more, 1 wt% or more, 3 wt% or more, 5 wt% or more, 10 wt% or more, 15 wt% or more, 20 wt% or more, 25 wt% or more, or 30 wt% or more, preferably 5 wt% or more, more preferably 10 wt% or more, further preferably 15 wt% or more. The amount of the inorganic nucleating agent in the composite particles may be 60 wt% or less, 50 wt% or less, 40 wt% or less, 35 wt% or less, 30 wt% or less, 25 wt% or less, 22.5 wt% or less, 20 wt% or less, 17.5 wt% or less, 15 wt% or less, 12.5 wt% or less, or 10 wt% or less, preferably 50 wt% or less, more preferably 45 wt% or less, further preferably 40 wt% or less. When the amount of the inorganic nucleating agent is within the range described above, the inside incorporation rate A can be enhanced, the grain size of the resulting composite particles can be refined, the shape of the particles can be made uniform, and the effects of the present disclosure such as spreading, adherence, finish and formability of the cosmetic can be well exhibited.

[Metal soap]

The metal soap may be a metal soap that is a salt of a fatty acid and at least one selected from the group consisting of calcium and magnesium. The metal soap may be a layer which coats at least a part of the inorganic nucleating agent.

The metal soap may be any of saturated fatty acid salts and unsaturated fatty acid salts, and may be any of linear and branched fatty acid salts. Further, a functional group such as a hydroxyl group, aldehyde group, epoxy group may be comprised in the fatty acid structure. Metal soaps include, for example, a caproic acid salt, heptylic acid salt, caprylic acid salt, capric acid salt, lauric acid salt, myristic acid salt, palmitic acid salt, stearic acid salt, arachic acid salt and behenic acid salt, and the like. These may be used singly or in combinations of two or more thereof. In one embodiment, at least one selected from a myristic acid salt, lauric acid salt, palmitic acid salt and stearic acid salt may be used as the metal soap.

Salts in the metal soap are a calcium salt and magnesium salt, and either one of these maybe used, or both of these may be used in combination.

The number of the carbon atoms of the fatty acid in the metal soap may be 6 or more, 8 or more, 10 or more, 12 or more, 14 or more, or 16 or more, preferably 8 or more, more preferably 12 or more. The number of the carbon atoms of the fatty acid in the metal soap may be 26 or less, 24 or less, 22 or less, 20 or less, 18 or less, 16 or less, or 14 or less, preferably 22or less, more preferably 20 or less. When the number of the carbon atoms of the fatty acid in the metal soap is within the above range, the effects of the present disclosure such as spreading, adherence, finish and formability of the cosmetic can be well exhibited.

(Amount of metal soap)

The amount of the metal soap in the composite particles may be 50 wt% or more, 60 wt% or more, 70 wt% or more, 80 wt% or more, 90 wt% or more, 95 wt% or more, or 99 wt% or more, or 99.9 wt% or more, preferably 50 wt% or more, more preferably 55 wt% or more, further preferably 60 wt% or more. The amount of the metal soap in the composite particles may be 99.9 wt% or less, 95 wt% or less, 90 wt% or less, 85 wt% or less, 80 wt% or less, 75 wt% or less, 70 wt% or less, or 65 wt% or less, preferably 95 wt% or less, 90 wt% or less, or 85 wt% or less. When the amount of the metal soap is within the above range, the inside incorporation rate A can be enhanced, the grain size of the resulting composite particles can be refined, the shape of the particles can be made uniform, and the effects of the present disclosure such as spreading, adherence, finish and formability of the cosmetic can be well exhibited.

[Other components]

Components other than the above components may be comprised in the composite particles in an amount within the range in which the effects of the present application would not be lost. Other components include an inorganic particle component other than the above, a metal soap component other than the above, a variety of other additive components and the like.

[Properties of composite particles]

The composite particles may have an inside incorporation rate A described below, a grain size summary value B, a degree of aggregation C, a loose bulk density D, and an average circularity E of the values within the particular ranges as described below.

(A: inside incorporation rate)

The composite particles in the present disclosure contain an inorganic nucleating agent and a metal soap, wherein the inorganic nucleating agent is incorporated in the inside of the composite particles. The composite particles preferably have the inside incorporation rate A within the particular range described below. The inside incorporation rate A is an indicator for expressing the extent to which the inorganic nucleating agent is localized in the inside of composite particles. Specifically, the inside incorporation rate A can be obtained by the following Formula (1).

$$\text{Inside incorporation rate } A = 100 - (X/X') \times 100$$
$$\text{Formula (1)}$$

[wherein,

X is the surface content of the inorganic nucleating agent in the composite particles,
X' is the whole content of the inorganic nucleating agent in the composite particles,
X and X' are average values obtained by conducting elemental analysis using SEM/EDX on 3 places within the area of 15 $\mu$m square under the condition of the acceleration voltage of 10 kV and the height of 15 mm for the composite particles before and after homogenization respectively.]

In the composite particles of the present disclosure, the inside incorporation rate A may be 25% or more, 30% or more, 40% or more, 50% or more, or 60% or more, preferably 40% or more, more preferably 50% or more, further preferably 60% or more. When the inside incorporation rate A is within the above range, the degree of contribution made by the inorganic nucleating agent to crystal shape and powder properties of the composite particles generated in a double decomposition reaction would be higher, thus desirable grain size summary value B, degree of aggregation C and an average circularity E are likely to be obtained.

Though the elements contained in the composite particles can be quantified by a scanning electron microscope/energy dispersive X-ray spectroscopy (SEM/EDX), the inorganic nucleating agent incorporated in the inside of the composite particles are not detected by SEM/EDX, therefore when the more inorganic nucleating agent are localized in the inside of the composite particles, the content of the inorganic nucleating agent in the composite particles would be calculated as the lower value compared the composite particles in which the inorganic nucleating agent is homogenized. That is, when the more inorganic nucleating agent are localized in the inside of the composite particles, the surface content X is lower compared to the whole content X', thus the inside incorporation rate would be higher value.

Homogenization may be pulverizing of composite particles. Similarly to preparation of a powder sample for EDX or IR etc., pulverization is conducted as finely as possible, so that the whole content of the inorganic nucleating agent in the composite particles may be as close to the true value as possible. For example, pulverization may be a process of pulverizing the composite particles to the size at which the particles can pass through a 325 mesh filter. The method of pulverization is not particularly limited, and for example, pulverization method using a mortar or a variety of milling apparatus can be employed.

(B: grain size summary value)

The composite particles used in the present disclosure preferably have a grain size summary value B within the particular range described below. A grain size summary value B is an indicator related to the variation of particle size. When the grain size summary value B is within the particular range, and the particles have the particular particle size distribution, further uniform covering is enabled in the case that the composite particles are used for the powder, and the effects of the present disclosure can be exhibited more stably. Specifically, a grain size summary value B can be obtained by the following Formula (2).

$$\text{Grain size summary value B} = (D90 - D10)/D50 \quad \text{Formula (2)}$$

D10: 10% cumulative diameter ($\mu$m) of composite particles on the basis of volume
D50: 50% cumulative diameter ($\mu$m) of composite particles on the basis of volume
D90: 90% cumulative diameter ($\mu$m) of composite particles on the basis of volume

The grain size summary value B may be 0.1 or more, 0.6 or more, 0.8 or more, 1 or more, 1.2 or more, 1.4 or more, or 1.4 or more, preferably 0.8 or more, more preferably 1 or more. The grain size summary value B may be 3 or less, 2.7 or less, 2.5 or less, 2.2 or less, 2 or less, 1.8 or less, 1.3 or less, 1 or less, or 0.8 or less, preferably 2.5 or less, more preferably 2.2 or less. When the grain size summary value B is within the above range, the variation of the particle size of the composite particles is lower, and the effects of the present disclosure such as spreading, adherence, finish and formability of the cosmetic can be well exhibited.

The 50% cumulative diameter of the composite particles on the basis of volume (D50) may be 1 $\mu$m or more, 3 $\mu$m or more, 5 $\mu$m or more, 10 $\mu$m or more, 30 $\mu$m or more, 50 $\mu$m or more, 75 $\mu$m or more, 100 $\mu$m or more, preferably 1 $\mu$m or more. The 50% cumulative diameter of the composite particles on the basis of volume (D50) may be 150 $\mu$m or less, 125 $\mu$m or less, 100 $\mu$m or less, 75 $\mu$m or less, 50 $\mu$m or less, 30 $\mu$m or less, 25 $\mu$m or less, or 20 $\mu$m or less, 15 $\mu$m or less, or 10 $\mu$m or less, preferably 30 $\mu$m or less. When the 50% cumulative diameter of the composite particles is within the above range, the effects of the present disclosure such as spreading, adherence, finish and formability of the cosmetic can be well exhibited. The grain size summary value B can be calculated using the particle size (cumulative diameter) measured by a laser diffraction method. The particle size distribution of the composite particles may be unimodal or multimodal, for example it is unimodal.

(C: degree of aggregation)

The composite particles used in the present disclosure preferably have the degree of aggregation C within the particular range described below. Degree of aggregation C is an indicator expressing how easily the composite particles disintegrate, and it quantifies the extent to which particles disintegrate finely, by applying vibration to the particles on a sieve without applying load, and determining the amount of particles remaining on the sieves of various mesh size. The degree of aggregation C is measured by a powder tester after leaving the particles under an environment of 80 °C for 10 minutes, and obtained by the following Formula (3).

Degree of aggregation C = {(mass of composite particles remaining on a sieve having mesh size of 350 $\mu$m)/2}$\times$100$\times$(1/1)+{(mass of composite particles remaining on a sieve having mesh size of 250 $\mu$m)/2}$\times$100$\times$(3/5)+{(mass of composite particles remaining on a sieve having mesh size of 150 $\mu$m)/2}$\times$100$\times$(1/5)} \quad Formula (3)

The degree of aggregation C may be 1% or more, 1.5 % or more, 2 % or more, 2.5% or more, 3% or more, 5 % or more, or 10 % or more, preferably 2 % or more. The degree of aggregation C may be 50 % or less, 40 % or less, 30 % or less, 25 % or less, 20 % or less, 15 % or less, or 10 % or less, preferably 25 % or less, more preferably 15 % or less. When the degree of aggregation C is within the above range, the effects of the present disclosure such as spreading, adherence, finish and formability of the cosmetic can be well exhibited.

The degree of aggregation C of the composite particles used herein and obtained by a powder tester is a value obtained by the following measurement method. That is, the following steps (a) to (f) are conducted sequentially using a powder tester for example (manufactured by HOSOKAWA MICRON CORPORATION, PT-N type).

(a) The composite particles to be measured are left in a thermostatic apparatus set at 80 °C for 10 minutes.
(b) Sieves having mesh size of 350 $\mu$m, 250 $\mu$m, 150 $\mu$m are placed in order from upper layer on a vibration table of the powder tester.
(c) 2.0 g of composite particles after the above step (a) are immediately put on the sieve having mesh size of 350 $\mu$m softly.
(d) The sieves are vibrated with the amplitude of 1 mm for 105 seconds.
(e) The mass of the composite particles remaining on each sieve is measured.
(f) The each mass obtained in the above step (e) is multiplied by weight of 1/1, 3/5, and 1/5 respectively, then added to each other to calculate the percentage value by the above Formula (3), and the obtained value is defined as the degree of aggregation C (%).

The above steps (a) to (f) are repeated 5 times, from which the average value is obtained and determined as the measured value.

Adjustment of the degree of aggregation C can be achieved by reacting a fatty acid alkaline metal salt with a source calcium salt or a source magnesium salt under a mild condition, and thus preventing aggregation between the composite particles in the slurry obtained by the reaction. That is, for example, adjustment can be achieved by conducting the reaction at a mild temperature at which the reaction rate is not decreased in the reaction of a fatty acid alkaline metal salt with a source calcium salt or a source magnesium salt, or by reducing aging time. By appropriately adjusting these factors in the reaction, the degree of aggregation C can be adjusted to the range specified in the present disclosure.

(D: loose bulk density)

The composite particles used in the present disclosure preferably have the loose bulk density D within the particular range described below. This enables high dispersibility to be obtained, and is likely to enable the effects of the present disclosure to be exhibited more stably. A loose bulk density D is obtained by the following Formula (4).

$$\text{Loose bulk density D = Mass of a cup containing a sample (g) / volume of a cup (cc)} \qquad \text{Formula (4)}$$

The loose bulk density D may be 0.08 or more, 0.1 or more, 0.12 or more, 0.135 or more, or 0.15 or more, preferably 0.12 or more, more preferably 0.135 or more. The loose bulk density D may be 0.25 or less, 0.22 or less, 0.20 or less, 0.18 or less, or 0.16 or less, preferably 0.2 or less, more preferably 0.18 or less. When the loose bulk density D is within the above range, high dispersibility is obtained, and the effects of the present disclosure such as spreading, adherence, finish and formability of the cosmetic can be well exhibited.

A loose bulk density D is a value obtained by the following measurement method. First, for example a powder tester (manufactured by HOSOKAWA MICRON CORPORATION, PT-N type) is used, a sieve having mesh size of 710 $\mu$m is placed on a vibration table, in which 250 cc of sample is charged, and vibration is applied for 30 seconds to collect fallen sample in a measurement cup placed below the sieve. After leveling off an excess of composite particles along the edge of the cup using an accessory blade, the mass of the cup containing the sample is measured. In the present disclosure, these operations and measurements are repeated 5 times, from which the average value is obtained and determined as the measured value of the bulk density D. In PT-N type, the measured value is indicated automatically.

(E: average circularity)

The composite particles used in the present disclosure preferably have the average circularity E (hereinafter, also referred to simply as "average circularity E") within the particular range described below for the particle group of 10 % particle size to 90 % particle size. The average circularity E in the present disclosure is an indicator for expressing the degree of irregularity of the particle shape of the composite particles, therefore it becomes closer to 1 when the surface shape of the composite particles is closer to a circular shape, and the average circularity E becomes a smaller value when the surface shape of the particles is more complicated. When the composite particles have an average circularity E within the particular range, dispersibility and covering property can be further enhanced, and the effects of the present disclosure are likely to be produced more stably. An average circularity E is used as a simple means for expressing the shape of the composite particles quantitatively, and can be defined as follows. First, particles having equivalent circle diameter within the range of 0.5 to 200 μm are measured using a flow-type particle image analyzer (for example, flow-type particle image analyzer "FPIA-3000" manufactured by SYSMEX CORPORATION), then the circularity (ai) of each particle measured in that step is obtained by the following Formula (5).

Circularity (ai) = (perimeter length of a circle having the same area as the projection image of the particle) / (perimeter length of the particle projection image)

Formula (5)

A flow-type particle image analyzer which can be used in the present disclosure, "FPIA-3000", uses the calculation method in which the circularity of each particle is calculated, the particles are classified into 61 classes that correspond to divided ranges of particle circularity of 0.4 to 1.0 based on the obtained circularity, and the average circularity is calculated using the center of each divided range and frequency.

In the present disclosure, the average circularity E for the particle group of 10 % particle size to 90 % particle size is the value obtained by dividing the sum of circularity of the particles within the range of 10 % particle size to 90 % particle size counting from smaller particle size of the particle size distribution among the all particles measured with the above measurement apparatus by the number of particles.

The average circularity E may be 0.7 or more, 0.75 or more, 0.8 or more, 0.85 or more, 0.9 or more, or 0.95 or more, preferably 0.75 or more, more preferably 0.85 or more. The average circularity E may be 1 or less, 0.95 or less, 0.9 or less, 0.85 or less, or 0.8 or less, preferably 0.95 or less, more preferably 0.9 or less. When the average circularity E is within the above range, dispersibility and covering property can be further enhanced, and the effects of the present disclosure such as spreading, adherence, finish and formability of the cosmetic can be well exhibited.

Detailed measurement procedure of the average circularity E is as follows for example. 30ml of ion-exchanged water from which solid impurities etc. have been removed in advance is provided in a container, then a surfactant as a dispersant, if necessary, preferably polyoxyethylene nonylphenyl ether (manufactured by NOF CORPORATION, product name: NONION NS-210) is added to the container, thereafter 20 mg of measurement sample is further added and dispersed uniformly. As a means for dispersing, for example an ultrasonic dispersing apparatus UH-50 type (manufactured by SMT CO., LTD., 20kHz, 50W) equipped with a titanium alloy tip having 5mm diameter as an oscillator is used, and dispersing treatment is conducted for 5 minutes to produce a dispersion for measurement having a dispersion concentration of 3,000 count/ml to 20,000 count/ml. In that process, the treatment is conducted while cooling the dispersion appropriately so that the temperature of the dispersion for measurement may not be 40 °C or more. After that, the average circularity E is obtained by conducting measurements using a flow-type particle image analyzer "FPIA-3000" and processing the obtained data.

Adjustment of the average circularity E can be achieved by reacting a fatty acid alkaline metal salt with a source calcium salt or a source magnesium salt under a mild condition, and preventing ununiform particle shape of the composite particles in the slurry obtained by the reaction. That is, for example, adjustment can be achieved by conducting the reaction at a mild temperature at which the reaction rate is not decreased in the reaction of a fatty acid alkaline metal salt with a source calcium salt or a source magnesium salt, or by slowing the dropping rate of a metal salt-containing aqueous solution into a fatty acid alkaline metal salt-containing aqueous solution, or by adding a polyalkylene glycol-based ether described above in order to stabilize the slurry. By appropriately adjusting these factors in the reaction, the average circularity E can be adjusted to the range specified in the present disclosure. Furthermore, it is more preferable that an inorganic nucleating agent be dispersed, with respect to the fatty acid alkaline metal salt aqueous solution, in the fatty acid alkaline metal salt-containing aqueous solution which has been subjected to adjustment, and then an aqueous solution containing a source calcium salt or a source magnesium salt be added thereto, or both be added to another reaction vessel. When a crystal nucleating agent is dispersed in a fatty acid alkaline metal salt-containing aqueous solution which is then reacted with a source calcium salt- or source magnesium salt-containing aqueous solution, composite particles are generated utilizing the crystal nucleating agent as nuclei, and thus the particle shape grow uniformly and high circularity is likely to be obtained.

[Amount of composite particles]

The amount of the composite particles in the cosmetic may be 1 wt% or more, 3 wt% or more, 5 wt% or more, 10 wt% or more, 20 wt% or more, 30 wt% or more, 40 wt% or more, 50 wt% or more, 60 wt% or more, or 70 wt% or more. The amount of the composite particles in the cosmetic may be 75 wt% or less, 65 wt% or less, 55 wt% or less, 45 wt% or less, 35 wt% or less, 25 wt% or less, or 15 wt% or less. When the amount of composite particles is within the above range, the

EP 4 506 037 A1

effects of the present disclosure such as spreading, adherence, finish and formability of the cosmetic can be well exhibited. Depending on the type of the cosmetic, the formulated amount of the composite particles may be appropriately varied.

{Method for manufacturing composite particles}

The composite particles of the present disclosure can be manufactured by reacting a fatty acid alkaline metal salt with a source calcium salt or source magnesium salt by double decomposition process in the presence of an inorganic nucleating agent.

A fatty acid alkaline metal salt is a salt of a fatty acid constituting the above-described metal soap and an alkaline metal (sodium, potassium, etc.).

The fatty acid alkaline metal salt used in the present disclosure may be obtained by reacting a fatty acid and a basic compound at a temperature which is generally higher than the melting point of the fatty acid, and at which the fatty acid does not decompose, preferably at 100 °C or lower, more preferably 50 to 100 °C, further preferably 60 to 95 °C, particularly preferably 70 to 95 °C. The basic compounds that can be source materials of the fatty acid alkaline metal salt include a hydroxide of an alkaline metal (sodium, potassium, etc.), and amines such as ammonia, monoethanolamine, diethanolamine and triethanolamine. The basic compounds are preferably hydroxides of an alkaline metal such as sodium and potassium (for example, sodium hydroxide, potassium hydroxide) in that the fatty acid alkaline metal salt formed from such hydroxide has high solubility in water. These basic compounds may be used alone or may be used in combination of two or more.

As a source calcium salt or source magnesium salt, a salt having high water solubility, for example, a salt having water solubility (20 °C) of 10 g/100 mL or more, 30 g/100 mL or more, 50 g/100 mL or more, or 70 g/100 mL or more is preferably selected, and chlorides, sulfates, nitrates etc. of calcium or magnesium such as calcium chloride, magnesium chloride, magnesium sulfate, calcium nitrate and magnesium nitrate may be mentioned. When the salt has high water solubility, it is preferable in terms of productivity since the salt at high concentration can react with the fatty acid alkaline metal salt.

The reaction of the fatty acid alkaline metal salt with the source calcium salt or source magnesium salt can be conducted by, for example, preparing the aqueous solution of a source calcium salt or source magnesium salt and the aqueous solution of the fatty acid alkaline metal salt separately, and subsequently mixing them. For example, mixing can be conducted by adding the aqueous solution of the source calcium salt or source magnesium salt to the aqueous solution of the fatty acid alkaline metal salt, or by adding both aqueous solutions to another reaction vessel.

With respect to mixing of the aqueous solution of the fatty acid alkaline metal salt and the aqueous solution of the source calcium salt or source magnesium salt, for example, when the whole aqueous solution of the source calcium salt or source magnesium salt is added at one time to the aqueous solution of the fatty acid alkaline metal salt, the resulting shape of the composite particles can be ununiform, and the particle size distribution can be broader. Therefore, in the present disclosure, it is preferable to add dropwise the aqueous solution of the source calcium salt or source magnesium salt little by little at an appropriate rate to the aqueous solution of the fatty acid alkaline metal salt.

In the above reaction, it is more preferable that the above-described inorganic nucleating agent be dispersed in the prepared aqueous solution of the fatty acid alkaline metal salt, then the aqueous solution of the source calcium salt or source magnesium salt be added thereto, or both be added to another reaction vessel.

An inorganic nucleating agent has the effect of promoting growth of the composite particles as nuclei, and of refining or making the particle size uniform. By adding the inorganic nucleating agent to the aqueous solution of the fatty acid alkaline metal salt, the fatty acid alkaline metal salt is dispersed uniformly, the inorganic nucleating agent serves as a nucleating agent for crystallization of the composite particles when the fatty acid alkaline metal salt is reacted with the aqueous solution of the source calcium salt or source magnesium salt, the particle shape is made uniform by nucleating agent as the starting point, and the particle size distribution of the composite particles becomes sharper.

The amount of the inorganic nucleating agent to be added may be 0.1 wt% or more, 0.5 wt% or more, 1 wt% or more, 3 wt% or more, 5 wt% or more, 10 wt% or more, or 15 wt% or more, preferably 0.5 wt% or more, more preferably 1 wt% or more relative to the total amount of the fatty acid alkaline metal salt and the inorganic nucleating agent. The amount of the inorganic nucleating agent to be added may be 30 wt% or less, 25 wt% or less, 22.5 wt% or less, 20 wt% or less, 17.5 wt% or less, 15 wt% or less, 12.5 wt% or less, or 10 wt% or less, preferably 25 wt% or less, more preferably 20 wt% or less relative to the total amount of the fatty acid alkaline metal salt and the inorganic nucleating agent. When the amount of the inorganic nucleating agent to be added is within the above range, the inside incorporation rate A is enhanced, the resulting grain size of the composite particles can be refined, the particle shape can be made uniform, and the effects of the present disclosure such as spreading, adherence, finish and formability of the cosmetic can be well exhibited.

The concentration of the fatty acid alkaline metal salt in the reaction aqueous solution may be 1 wt% or more, 5 wt% or more, 10 wt% or more, or 15 wt% or more, preferably 5 wt% or more. The concentration of the fatty acid alkaline metal salt in the reaction aqueous solution may be 50 wt% or less, 40 wt% or less, 30 wt% or less, 20 wt% or less, or 10 wt% or less, preferably 15 wt% or less. It is preferable that the concentration be within the above range, since more uniform reaction can be provided while maintaining high productivity of the composite particles.

The concentration of the source calcium salt or source magnesium salt in the aqueous solution of the source calcium salt or source magnesium salt may be 10 wt% or more, 20 wt% or more, or 30 wt% or more. The concentration of the source calcium salt or source magnesium salt in the aqueous solution of the source calcium salt or source magnesium salt may be 60 wt% or less, 50 wt% or less, 40 wt% or less, or 30 wt% or less. The above range is suitable in terms of productivity of the composite particles, and handling property of the aqueous solution of the fatty acid alkaline metal salt or the resulting slurry of the composite particles.

The reaction of the fatty acid alkaline metal salt with the source calcium salt or source magnesium salt is conducted under the reaction temperature condition that the person skilled in the art usually employ considering the solubility of the fatty acid alkaline metal salt. The reaction temperature may be 20 °C or more, 40 °C or more, 60 °C or more, or 80 °C or more, preferably 60 °C or more. The reaction temperature may be the boiling point or less, for example 120 °C or less, 110 °C or less, 100 °C or less, 90 °C or less, 80 °C or less, or 70 °C or less, preferably 100 °C or less. When the reaction temperature is within the above range, satisfactory reaction rate can be achieved.

In the reaction of the fatty acid alkaline metal salt with the source calcium salt or source magnesium salt, a surfactant may exist for the purpose of stabilizing the slurry and enhancing productivity of the composite particles. Nonionic surfactants, for example polyalkylene glycol-based ethers, in particular triblock ethers having a structure in which oxypropylene block (PO) is interposed between oxyethylene blocks (EO) (EO-PO-EO) is preferably used as a surfactant. The content of the surfactant may be 0.01 wt% or more, 0.1 wt% or more, 0.3 wt% or more, 0.5 wt% or more, or 1 wt% or more relative to the fatty acid alkaline metal salt. The content of the surfactant may be 5 wt% or less, 3 wt% or less, 1 wt% or less, 0.5 wt% or less, or 0.1 wt% or less relative to the fatty acid alkaline metal salt.

The surfactant may exist in the reaction system before reacting the basic compound with the fatty acid, and may exist in the reaction system before reacting the fatty acid alkaline metal salt with the source calcium salt or source magnesium salt.

The composite particle slurry is obtained by the above method. Without being treated, or after removing solvent by a centrifugal extractor, filter press, vacuum rotary filter etc., and if necessary washing and removing salts containing calcium or magnesium generated as byproducts, this composite particle slurry is dried by a rotary dryer, pneumatic conveying dryer, ventilating dryer, spray dryer, fluidized bed dryer, and the like. The drying process may be any one of continuous type or batch type, or under normal pressure or under vacuum. Further, the dried composite particles are pulverized if necessary. The pulverizing process is not particularly limited, and for example pulverizing process using a pin mill, jet mill, atomizer mill etc. can be employed. The pulverized composite particles are classified. That is, classification is conducted using a multistage sieving device which performs sieving by applying vibration, and thus the particle size distribution is adjusted. The composite particles of the present disclosure can be obtained in this way.

{Oily component}

The cosmetic of the present disclosure may contain an oily component. The oily component may be a liquid oil, paste oil or solid oil, preferably a liquid oil or paste oil. The composite particles of the present disclosure are not contained in the oily component.

The oily component may be those used in a common cosmetic, and the examples of the oily component include linear or branched hydrocarbon oils such as liquid paraffin, light isoparaffin, liquid isoparaffin, vaseline, polybutene, polyisobutene, hydrogenated polyisobutene, hydrogenated polydecene, squalane and squalene; vegetable oils such as avocado oil, macadamia nut oil, olive oil, rapeseed oil, sesame oil, wheat germ oil, linseed oil, cottonseed oil, soybean oil, palm oil, coconut oil, castor oil, jojoba oil, sunflower oil, camellia oil and corn oil; animal oils such as lanolin, liquid lanolin, whale oil and shark liver oil; ester oils such as isopropyl myristate, octyldodecyl myristate, isopropyl isostearate, isononyl isononanoate, butyl stearate, oleyl oleate, isotridecyl isononanoate, isostearyl myristate, octyldodecyl ricinoleate, octyl hydroxystearate, ethylhexyl palmitate, cetyl ethylhexanoate, octyl methoxycinnamate, tocopherol acetate, propylene carbonate, propylene glycol dicaprylate, neopentyl glycol dicaprate, neopentyl glycol diethylhexanoate, propanediol diisostearate, glyceryl monoisostearate monomyristate, propanediol dicaprate/dicaprylate, glyceryl tricaprylate / tricaprate, glyceryl tri(2-ethylhexanoate), triethylhexanoin, trimethylolpropane tri(2-ethylhexanoate), trimethylolpropane triisostearate, pentaerythrityl tetraoctanoate, pentaerythritol tetraethylhexanoate, polyglyceryl-6 octacaprylate, ditrimethylolpropane (isostearate/sebacate) oligoester, dipentaerythrityl tripolyhydroxystearate, di(phytosteryl/octyldodecyl) lauroyl glutamate, di(phytosteryl/behenyl/octyldodecyl) lauroyl glutamate, trehalose isostearate esters, ethylhexyl hydroxystearate, phytosterol fatty acid esters, cholesterol fatty acid esters, polyglycerin fatty acid esters, pentaerythritol fatty acid esters, trilanolin fatty acid glyceryl, branched or hydroxylated fatty acid cholesteryl, dipentaerythritol fatty acid esters (dipentaerythritol hexaoxystearate), hydrogenated castor oil isostearate, hydrogenated castor oil monohydroxy stearate, caprylic/capryic/myristic/stearic triglyceride, hydrogenated castor oil dimer dilinoleate, (phytosteryl/isostearyl/cetyl/stearyl/behenyl) dimer dilinoleate, phytosteryl oleate, dl-α-tocopherol, dl-α-tocopheryl nicotinate and myristyl lactate; higher alcohols such as octyl dodecanol, myristyl alcohol, stearyl alcohol, behenyl alcohol and isostearyl alcohol; fatty acids such as lauric acid, myristyc acid, palmitic acid, stearic acid, oleic acid, isostearic acid, soft lanolin fatty acid; silicone oils such as diphenyl dimethicone, dimethyl polysiloxane, methyl cyclopolysiloxane, methylphenyl polysiloxane, methylhydrogen polysiloxane, higher alcohol-modified organopolysiloxane, tris (trimethylsiloxy) methylsilane and trimethyl pentaphenyl trisiloxane; fluorine oils such as fluoropolyether and perfluoroalkylether silicone; waxes such as beeswax, candelilla wax, carnauba wax and jojoba wax. These may be used singly or in combinations of two or more thereof.

[Amount of oily component]

The amount of the oily component in the cosmetic may be 0.1 wt% or more, 0.5 wt% or more, 1 wt% or more, 3 wt% or more, 5 wt% or more, or 10 wt% or more. The amount of the oily component in the cosmetic may be 20 wt% or less, 15 wt% or less, 10 wt% or less, 5 wt% or less, or 3 wt% or less. When the amount of the oily component is within the above range, the effects of the present disclosure such as spreading, adherence, finish and formability can be well exhibited in the cosmetic having the oily component.

{Spherical particles}

The cosmetic in the present disclosure may contain spherical particles. The spherical particles may be inorganic particles or organic particles, preferably silica particles or silicone particles. The spherical inorganic particles or spherical organic particles are appropriately used in the solid powder cosmetics (for example, powder foundation).

Examples of the spherical particles include spherical inorganic particles such as spherical silica, spherical calcium carbonate, spherical alumina, spherical titania and spherical zirconia; spherical organic particles such as spherical cellulose, spherical silicone particles (for example, (vinyldimethicone/methicone silsesquioxane) crosspolymer etc.), spherical silk powder, spherical nylon particles, spherical acrylic particles, spherical polyethylene particles, spherical polypropylene particles, spherical polystyrene particles, spherical nylon particles, spherical urethane particles and spherical starch particles. These may be used singly or in combinations of two or more thereof. Also, the spherical particles can be subjected to hydrophilization treatment or hydrophobization treatment to be adopted.

The cumulative diameter (D50) of the spherical particles on the basis of the volume may be 1 $\mu$m or more, 3 $\mu$m or more, 5 $\mu$m or more, 10 $\mu$m or more, 30 $\mu$m or more, 50 $\mu$m or more, 75 $\mu$m or more, 100 $\mu$m or more. The cumulative diameter (D50) of the spherical particles on the basis of the volume may be 150 $\mu$m or less, 125 $\mu$m or less, 100 $\mu$m or less, 75 $\mu$m or less, 50 $\mu$m or less, 30 $\mu$m or less, 25 $\mu$m or less, or 20 $\mu$m or less, 15 $\mu$m or less, or 10 $\mu$m or less, preferably 30 $\mu$m or less. When the cumulative diameter of the spherical particles is within the above range, the effects of the present disclosure such as spreading, adherence, finish and formability of the cosmetic can be well exhibited. The 50% cumulative diameter (D50) can be measured by a laser diffraction method. The particle size distribution of the spherical particles may be unimodal or multimodal, for example it is unimodal.

[Amount of spherical particles]

The amount of the spherical particles in the cosmetic may be 1 wt% or more, 3 wt% or more, 5 wt% or more, 10 wt% or more, 15 wt% or more, or 20 wt% or more. The amount of the spherical particles in the cosmetic may be 50 wt% or less, 40 wt% or less, 30 wt% or less, 20 wt% or less, or 10 wt% or less. The amount of the spherical particles is within the above range, the effects of the present disclosure such as spreading, adherence, finish and formability of the cosmetic can be well exhibited.

{Other components]

Furthermore, the cosmetic of the present disclosure can contain components commonly used in cosmetics as other components, for example, surfactant, extender pigment, colorants, ultraviolet protective agent, oil soluble gelling agent (organic modified clay mineral), water soluble polymer, resin, antiperspirant, moisturizing agent, antibacterial-antiseptic agent, fragrance, salts, antioxidizing agent, pH adjusting agent, chelating agent, refreshing agent, anti-inflammatory agent, skin-beautifying ingredient (skin-lightening agent, cell activator, skin damage improving agent, blood circulation promoter, skin astringent, antiseborrheic agent etc.), vitamins, amino acids and the like in an amount within the range in which the effects of the present disclosure would not be lost. These may be used singly or in combinations of two or more thereof.

The surfactant includes anionic, cationic, nonionic or amphoteric surfactant, and a silicone-based or hydrocarbon-based surfactant is included. These may be used singly or in combinations of two or more thereof.

Examples of the extender pigment include mica, synthetic mica, sericite, synthetic sericite, talc, kaolin, silicon carbide, silicate, barium sulfate, hydroxyapatite, bentonite, smectite, aluminum oxide, silica, magnesium oxide, zirconium oxide, magnesium carbonate, calcium carbonate, chromium oxide, aluminum hydroxide, magnesium hydroxide, boron nitride, hydroxyapatite, alumina, lauroyl lysine, metal soap powder and the like. These may be used singly or in combinations of two or more thereof.

Examples of the colorant include inorganic white pigments such as titanium oxide and zinc oxide; inorganic colored pigments such as yellow iron oxide, red iron oxide, black iron oxide, carbon black, ultramarine, Prussian blue, Prussian blue titanium oxide, black titanium oxide, chromium oxide, chromium hydroxide and sintered titanium / sintered titanium oxide; organic pigments such as Red No. 201, Red No. 202, Red No. 226, Yellow No. 401, Blue No. 404, etc.; lake pigments such as Red No. 104, Red No. 230, Yellow No. 4, Yellow No. 5, Blue No. 1, etc.; and those obtained by coating organic pigments with a polymer such as polymethacrylate ester, and the like. Pearl pigments include mica titanium, colcothar-coated (titanium oxide/aluminum hydroxide) mixture, colcothar -coated mica, bismuth oxychloride, titanium oxide-coated bismuth oxychloride, iron oxide-coated mica titanium, organic pigment-coated mica titanium, silicic acid/- titaniumtreated mica, titanium oxide-coated talc, silicon dioxide/colcothar -treated aluminum, inorganic powders such as titanium oxide-coated glass powder, resin-coated plate-like aluminum powder, titanium oxide - yellow iron oxide - colcothar/lauryl methacrylate - ethylene glycol dimethacrylate copolymer mixture, and the like. These may be used singly or in combinations of two or more thereof.

Examples of the ultraviolet protective agent include ultraviolet absorbing agents such as ethylhexyl methoxycinnamate, octocrylene, dimethicodiethyl benzalmalonate, polysilicone-15, t-butyl methoxybenzoylmethane, ethylhexyl triazone, diethylamino hydroxybenzoyl hexyl benzoate, bis-ethylhexyloxyphenol methoxyphenyl triazine, oxybenzone-3, methylene bis-benzotriazolyl tetramethylbutylphenol, phenylbenzimidazole sulfonic acid, homosalate and ethylhexyl salicylate; metal oxide fine particle such as titanium oxide fine particle, zinc oxide fine particle, iron oxide fine particle and cerium oxide fine particle. These may be used singly or in combinations of two or more thereof.

Examples of the oil soluble gelling agent include metal soaps such as aluminum stearate, magnesium stearate, zinc myristate; amino acid derivatives such as N-lauroyl-L-glutamic acid dibutylamide; dextrin fatty acid esters such as dextrin palmitate ester, dextrin stearate ester and dextrin 2-ethylhexanoate palmitate ester; sucrose fatty acid esters such as sucrose palmitate ester and sucrose stearate ester; fructo-oligosaccharide fatty acid esters such as fructo-oligosaccharide stearate ester and fructo-oligosaccharide 2-ethyl hexanoate ester; benzylidene derivatives of sorbitol such as monobenzylidene sorbitol and dibenzylidene sorbitol; organic modified clay minerals such as dimethylbenzyl dodecylammonium montmorillonite clay and dimethyl dioctadecylammmonium montmorillonite clay. These may be used singly or in combinations of two or more thereof. These may be used singly or in combinations of two or more thereof.

Examples of the water soluble polymer include carboxy vinyl polymer, ammonium polyacrylate, sodium polyacrylate, sodium acrylate/alkyl acrylate/sodium methacrylate/alkyl methacrylate copolymer, carrageenan, pectin, mannan, curdlan, chondroitin sulfate, starch, glycogen, gum arabic, sodium hyaluronate, tragacanth gum, xanthan gum, mucoitinsulfuric acid, hydroxyethyl guar gum, carboxymethyl guar gum, guar gum, dextrin, keratosulfate, locust bean gum, succinoglucan, chitin, chitosan, carboxymethyl chitin, agar and the like. These may be used singly or in combinations of two or more thereof.

Examples of the antiperspirant include one or more antiperspirants selected from, for example, aluminum chlorohydrate, aluminum chloride, aluminum sesquichlorohydrate, zirconyl hydroxychloride, aluminum zirconium hydroxychloride, aluminum zirconium glycine complex and the like. These may be used singly or in combinations of two or more thereof.

Examples of the moisturizing agent include one or more moisturizing agents selected from, for example, glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methylglucoside, polyoxypropylene methylglucoside and the like. These may be used singly or in combinations of two or more thereof.

Examples of the antibacterial-antiseptic agent include alkyl p-hydroxybenzoate ester, benzoic acid, sodium benzoate, sorbic acid, potassium sorbate and phenoxyethanol, and the antibacterial agent includes benzoic acid, salicylic acid, carbolic acid, sorbic acid, alkyl p-hydroxybenzoate ester, p-chloro-m-cresol, hexachlorophen, benzalkonium chloride, chlorhexidine chloride, trichlorocarbanilide, photosensitive dye, phenoxyethanol and the like. These may be used singly or in combinations of two or more thereof.

Examples of the salt include, for example, an inorganic salt, organic acid salt, amine salt and amino acid salt etc. The inorganic salt includes a sodium salt, potassium salt, magnesium salt, calcium salt, aluminum salt, zirconium salt, zinc salt of inorganic acids such as hydrochloric acid, sulfuric acid, carbonic acid, nitric acid and the like; the organic acid salt includes a salt of an organic acid such as, for example, acetic acid, dehydroacetic acid, citric acid, malic acid, succinic acid, ascorbic acid, stearic acid and the like; the amine salt and amino acid salt include, for example, a salt of an amine such as triethanolamine, and a salt of amino acid such as glutamic acid. Besides, salts of hyaluronic acid, chondroitin sulfate and the like, aluminum zirconium glycine complex and the like, and in addition, neutral salts of acid-alkali used in cosmetic formulations can be also used. These may be used singly or in combinations of two or more thereof.

The oxidizing agent includes tocopherol, butyl hydroxyanisole, dibutyl hydroxytoluene, phytic acid and the like, the pH adjusting agent includes lactic acid, citric acid, glycolic acid, succinic acid, tartaric acid, dl-malic acid, potassium carbonate, sodium hydrogen carbonate, sodium hydrogen ammonium and the like, the chelating agent includes alanine, sodium edetate salt, sodium polyphosphate, sodium metaphosphate, phosphoric acid and the like, the refreshing agent includes L-menthol, camphor and the like, the anti-inflammatory agent includes allantoin, glycyrrhizic acid and a salt thereof, glycyrrhetinic acid and stearyl glycyrrhetinate, tranexamic acid, azulene and the like. These may be used singly or in combinations of two or more thereof.

The skin-beautifying ingredient includes, for example, skin-lightening agents such as placenta extract, arbutin, glutathione and Saxifraga Sarmentosa Extract; cell activators such as royal jelly, photosensitive dye, cholesterol derivatives and calf blood extract; skin damage improving agent; blood circulation promoters such as 4-Hydroxy-3-methoxybenzyl Nonylic Acid Amide, benzyl nicotinate ester, $\beta$-butoxyethyl nicotinate ester, capsaicin, zingeron, cantharides tincture, Ichthammol, caffeine, tannic acid, $\alpha$-borneol, tocopheryl nicotinate, inositol hexanicotinate, cyclandelate, cinnarizine, tolazoline, acetylcholine, verapamil, cepharanthine, $\gamma$-orizanol; skin astringents such as zinc oxide, tannic acid; antiseborrheic agents such as sulfur, thianthol, and the like. These may be used singly or in combinations of two or more thereof.

The vitamins include, for example, vitamin A such as vitamin A oil, retinol, retinyl acetate and retinyl palmitate; vitamin B2 such as riboflavin, riboflavin butyrate and flavine adenine nucleotide; vitamin B6 such as pyridoxine hydrochloride, pyridoxine dioctanoate, pyridoxine tripalmitate; vitamin B such as vitamin B12 and a derivative thereof, vitamin B15 and a derivative thereof; vitamin C such as L-ascorbic acid, L-ascorbyl dipalmitate ester, sodium L-ascorbic acid-2-sulfate, L-ascorbyl phosphate diester dipotassium; vitamin D such as ergocalciferol and cholecalciferol; vitamin E such as $\alpha$-tocopherol, $\beta$-tocopherol, $\gamma$-tocopherol, dl-$\alpha$-tocopherol acetate, dl-$\alpha$-tocopherolnicotinate and dl-$\alpha$-tocopherol succi-nate; nicotinic acids such as vitamin H, vitamin P, nicotinic acid, benzyl nicotinate and nicotinamide; pantothenic acids such as calcium pantothenate, D-pantothenyl alcohol, pantothenyl ethyl ether, acetyl pantothenyl ethyl ether; biotin and the like. These may be used singly or in combinations of two or more thereof.

The amino acids include, for example, glycine, valine, leucine, isoleucine, Serine, Threonine, Phenylalanine, arginine, lysine, aspartic acid, glutamic acid, cystine, cysteine, Methionine, tryptophan and the like, and nucleic acids include deoxyribonucleic acid and the like, hormones include estradiol, ethenyl estradiol and the like. These may be used singly or in combinations of two or more thereof.

<Method for manufacturing the cosmetic>

The method for manufacturing the cosmetic in the present disclosure is not particularly limited, and a known manufacturing method can be used. For example, the method can be mentioned in which raw materials are stirred so that the raw materials may be dispersed uniformly while being heated if necessary. If desired, a high dispersion machine such as a roller mill, high pressure homogenizer, bead mill, microfluidizer etc. may be used. The cosmetic may be molded, and molding may be dry molding or wet molding.

<Application of the cosmetic>

Application of the cosmetic in the present disclosure includes cosmetics for skin care such as milky lotion, cream, cleansing product, pack cosmetic, massage cosmetic, beauty liquid, beauty oil, washing agent, hand cream, lip cream, wrinkle masking agent, body powder, essence and shaving lotion; cosmetics for makeup such as powder foundation, liquid foundation, makeup base, eye shadow, concealer, face powder, lipstick and mascara; cosmetics for hair such as shampoo, hair conditioner, hair treatment agent, cream for scalp and hair setting agent; antiperspirants such as deodorant agent; ultraviolet protective agents such as sunscreen oil, sunscreen milky lotion and sunscreen cream, and the like. The cosmetic of the present disclosure has good spreading, adherence, finish and formability etc., and is also suitably used as a cosmetic in liquid form or paste form, in particular suitable for a solid powder cosmetic. The solid powder cosmetic is a cosmetic composed mainly of powder, and may be molded (for example, dry molding or wet molding).

According to the present disclosure, a novel cosmetic can be provided that exhibits an excellent spreading when applied, also excellent adherence after application and excellent finish after application, as well as good feeling of use. According to the present disclosure, preferably a novel cosmetic can be provided that further exhibits excellent formability, anti-caking, rubbing-off property and/or feeling of finish, as well as good feeling of use.

Though the embodiments are described as stated above, it will be understood that various modifications in modes and details can be made without departing from the scope and spirit of the claims.

Examples

The present disclosure will be described in detail hereinafter by providing Examples, however the present disclosure is not limited to these Examples.

<Synthesis of composite particle>

{Synthesis Example 1}

200 g of myristic acid (NAA-142, manufactured by NOF CORPORATION) and 2119 g of water were charged into a 3L separable flask, and heated to 80 °C. Then, 73.6 g of 48 mass% sodium hydroxide aqueous solution was added to the flask, and the mixture was stirred at the same temperature (80 °C) for 1 hour to obtain the aqueous solution of the fatty acid alkaline metal salt. 43.5 g of borosilicate glass (Ca/Al) (FTD008FY manufactured by Nippon Sheet Glass Co., Ltd.) having an average particle size of 8.2 μm was added to the obtained solution, which was then stirred for 1 hour. Thereafter, 145.5 g of 35 mass% calcium chloride aqueous solution was added dropwise to the aqueous solution of the fatty acid alkaline metal salt over a period of 30 minutes while maintaining the temperature at 80 °C. After completing dropwise addition, the mixture was stirred for 30 minutes while maintaining the temperature at 80 °C for aging. 1500 g of water was added to the resulted slurry of the fatty acid calcium salt aqueous solution, which was then cooled to 65°C or lower. Thereafter, the slurry was filtered by a suction filer, washed with 1000 g of water 2 times, and the resulted cake was dried with a micron dryer, pulverized and classified to obtain the calcium myristate salt particles having borosilicate glass (Ca/Al) as a nucleating agent.

{Synthesis Example 2}

200 g of lauric acid (NAA-122, manufactured by NOF CORPORATION) and 2136 g of water were charged into a 3L separable flask, and heated to 80 °C. Then, 84.0 g of 48 mass% sodium hydroxide aqueous solution was added to the flask, and the mixture was stirred at the same temperature (80 °C) for 1 hour to obtain the aqueous solution of the fatty acid alkaline metal salt. 44.0 g of borosilicate glass (Ca/Al) (FTD008FY manufactured by Nippon Sheet Glass Co., Ltd.) having an average particle size of 8.2 μm was added to the obtained solution, which was then stirred for 1 hour. Thereafter, 166.2 g of 35 mass% calcium chloride aqueous solution was added dropwise to the aqueous solution of the fatty acid alkaline metal salt over a period of 30 minutes while maintaining the temperature at 80 °C. After completing dropwise addition, the mixture was stirred for 30 minutes while maintaining at 80 °C for aging. 1500 g of water was added to the resulted slurry of the fatty acid calcium salt aqueous solution, which was then cooled to 65°C or lower. Thereafter, the slurry was filtered by a suction filer, washed with 1000 g of water 2 times, and the resulted cake was dried with a micron dryer, pulverized and classified to obtain the calcium laurate salt particles having borosilicate glass (Ca/Al) as a nucleating agent.

{Synthesis Example 3}

200 g of palmitic acid (NAA-160, manufactured by NOF CORPORATION) and 2106 g of water were charged into a 3L separable flask, and heated to 80 °C. Then, 65.6 g of 48 mass% sodium hydroxide aqueous solution was added to the flask, and the mixture was stirred at the same temperature (80 °C) for 1 hour to obtain the aqueous solution of the fatty acid alkaline metal salt. 43.1 g of borosilicate glass (Ca/Al) (FTD008FY manufactured by Nippon Sheet Glass Co., Ltd.) having an average particle size of 8.2 μm was added to the obtained solution, which was then stirred for 1 hour. Thereafter, 129.8 g of 35 mass% calcium chloride aqueous solution was added dropwise to the aqueous solution of the fatty acid alkaline metal salt over a period of 30 minutes while maintaining the temperature at 80 °C. After completing dropwise addition, the mixture was stirred for 30 minutes while maintaining at 80 °C for aging. 1500 g of water was added to the resulted slurry of the fatty acid calcium salt aqueous solution, which was then cooled to 65°C or lower. Thereafter, the slurry was filtered by a suction filer, washed with 1000 g of water 2 times, and the resulted cake was dried with a micron dryer, pulverized and classified to obtain the calcium palmitate salt particles having borosilicate glass (Ca/Al) as a nucleating agent.

{Synthesis Example 4}

200 g of stearic acid (NAA-180, manufactured by NOF CORPORATION) and 2095 g of water were charged into a 3L separable flask, and heated to 80 °C. Then, 59.1 g of 48 mass% sodium hydroxide aqueous solution was added to the flask, and the mixture was stirred at the same temperature (80 °C) for 1 hour to obtain the aqueous solution of the fatty acid alkaline metal salt. 42.8 g of borosilicate glass (Ca/Al) (FTD008FY manufactured by Nippon Sheet Glass Co., Ltd.) having an average particle size of 8.2 μm was added to the obtained solution, which was then stirred for 1 hour. Thereafter, 116.8 g of 35 mass% calcium chloride aqueous solution was added dropwise to the aqueous solution of the fatty acid alkaline metal salt over a period of 30 minutes while maintaining the temperature at 80 °C. After completing dropwise addition, the mixture was stirred for 30 minutes while maintaining at 80 °C for aging. 1500 g of water was added to the resulted slurry of the fatty acid calcium salt aqueous solution, which was then cooled to 65°C or lower. Thereafter, the slurry was filtered by a suction filer, washed with 1000 g of water 2 times, and the resulted cake was dried with a micron dryer, pulverized and classified to obtain the calcium stearate salt particles having borosilicate glass (Ca/Al) as a nucleating agent.

{Synthesis Example 5}

200 g of myristic acid (NAA-142, manufactured by NOF CORPORATION) and 2119 g of water were charged into a 3L separable flask, and heated to 80 °C. Then, 73.6 g of 48 mass% sodium hydroxide aqueous solution was added to the flask, and the mixture was stirred at the same temperature (80 °C) for 1 hour to obtain the aqueous solution of the fatty acid alkaline metal salt. 43.5 g of barium sulfate (plate-like barium sulfate H manufactured by SAKAI CHEMICAL INDUSTRY CO., LTD.) having an average particle size of 8.1 $\mu$m was added to the obtained solution, which was then stirred for 1 hour. Thereafter, 145.5 g of 35 mass% calcium chloride aqueous solution was added dropwise to the aqueous solution of the fatty acid alkaline metal salt over a period of 30 minutes while maintaining the temperature at 80 °C. After completing dropwise addition, the mixture was stirred for 30 minutes while maintaining at 80 °C for aging. 1500 g of water was added to the resulted slurry of the fatty acid calcium salt aqueous solution, which was then cooled to 65°C or lower. Thereafter, the slurry was filtered by a suction filer, washed with 1000 g of water 2 times, and the resulted cake was dried with a micron dryer, pulverized and classified to obtain the calcium myristate salt particles having barium sulfate as a nucleating agent.

{Synthesis Example 6}

200 g of myristic acid (NAA-142, manufactured by NOF CORPORATION) and 2119 g of water were charged into a 3L separable flask, and heated to 80 °C. Then, 73.6 g of 48 mass% sodium hydroxide aqueous solution was added to the flask, and the mixture was stirred at the same temperature (80 °C) for 1 hour to obtain the aqueous solution of the fatty acid alkaline metal salt. 42.1 g of borosilicate glass (Ca/Al) (FTD008FY manufactured by Nippon Sheet Glass Co., Ltd.) having an average particle size of 8.2 $\mu$m was added to the obtained solution, which was then stirred for 1 hour. Thereafter, 124.9 g of 35 mass% magnesium chloride aqueous solution was added dropwise to the aqueous solution of the fatty acid alkaline metal salt over a period of 30 minutes while maintaining the temperature at 80 $^0$C. After completing dropwise addition, the mixture was stirred for 30 minutes while maintaining at 80 $^0$C for aging. 1500 g of water was added to the resulted slurry of the fatty acid calcium salt aqueous solution, which was then cooled to 65$^0$C or lower. Thereafter, the slurry was filtered by a suction filer, washed with 1000 g of water 2 times, and the resulted cake was dried with a micron dryer, pulverized and classified to obtain the magnesium myristate salt particles having borosilicate glass (Ca/Al) as a nucleating agent.

{Synthesis Example 7}

200 g of myristic acid (NAA-142, manufactured by NOF CORPORATION) and 2119 g of water were charged into a 3L separable flask, and heated to 80 $^0$C. Then, 73.6 g of 48 mass% sodium hydroxide aqueous solution was added to the flask, and the mixture was stirred at the same temperature (80 $^0$C) for 1 hour to obtain the aqueous solution of the fatty acid alkaline metal salt. 42.1 g of barium sulfate (plate-like barium sulfate H manufactured by SAKAI CHEMICAL INDUSTRY CO., LTD.) having an average particle size of 8.1 $\mu$m was added to the obtained solution, which was then stirred for 1 hour. Thereafter, 124.9 g of 35 mass% magnesium chloride aqueous solution was added dropwise to the aqueous solution of the fatty acid alkaline metal salt over a period of 30 minutes while maintaining the temperature at 80 $^0$C. After completing dropwise addition, the mixture was stirred for 30 minutes while maintaining at 80 $^0$C for aging. 1500 g of water was added to the resulted slurry of the fatty acid calcium salt aqueous solution, which was then cooled to 65$^0$C or lower. Thereafter, the slurry was filtered by a suction filer, washed with 1000 g of water 2 times, and the resulted cake was dried with a micron dryer, pulverized and classified to obtain the magnesium myristate salt particles having barium sulfate as a nucleating agent.

{Synthesis Example 8}

200 g of myristic acid (NAA-142, manufactured by NOF CORPORATION) and 2119 g of water were charged into a 3L separable flask, and heated to 80 $^0$C. Then, 73.6 g of 48 mass% sodium hydroxide aqueous solution was added to the flask, and the mixture was stirred at the same temperature (80 $^0$C) for 1 hour to obtain the aqueous solution of the fatty acid alkaline metal salt. 43.5 g of talc (JA-46R manufactured by ASADA MILLING CO., LTD.) having an average particle size of 11.2 $\mu$m was added to the obtained solution, which was then stirred for 1 hour. Thereafter, 145.5 g of 35 mass% calcium chloride aqueous solution was added dropwise to the aqueous solution of the fatty acid alkaline metal salt over a period of 30 minutes while maintaining the temperature at 80 $^0$C. After completing dropwise addition, the mixture was stirred for 30 minutes while maintaining at 80 $^0$C for aging. 1500 g of water was added to the resulted slurry of the fatty acid calcium salt aqueous solution, which was then cooled to 65$^0$C or lower. Thereafter, the slurry was filtered by a suction filer, washed with 1000 g of water 2 times, and the resulted cake was dried with a micron dryer, pulverized and classified to obtain the calcium myristate salt particles having talc as a nucleating agent.

{Synthesis Example 9}

200 g of myristic acid (NAA-142, manufactured by NOF CORPORATION) and 2119 g of water were charged into a 3L separable flask, and heated to 80 $^0$C. Then, 73.6 g of 48 mass% sodium hydroxide aqueous solution was added to the flask, and the mixture was stirred at the same temperature (80 $^0$C) for 1 hour to obtain the aqueous solution of the fatty acid alkaline metal salt. 43.5 g of boron nitride (SHP-5 manufactured by Mizushima Ferroalloy Co., Ltd.) having an average particle size of 25.4 $\mu$m was added to the obtained solution, which was then stirred for 1 hour. Thereafter, 145.5 g of 35 mass% calcium chloride aqueous solution was added dropwise to the aqueous solution of the fatty acid alkaline metal salt over a period of 30 minutes while maintaining the temperature at 80 $^0$C. After completing dropwise addition, the mixture was stirred for 30 minutes while maintaining at 80 $^0$C for aging. 1500 g of water was added to the resulted slurry of the fatty acid calcium salt aqueous solution, which was then cooled to 65$^0$C or lower. Thereafter, the slurry was filtered by a suction filer, washed with 1000 g of water 2 times, and the resulted cake was dried with a micron dryer, pulverized and classified to obtain the calcium myristate salt particles having boron nitride as a nucleating agent.

{Synthesis Example 10}

200 g of myristic acid (NAA-142, manufactured by NOF CORPORATION) and 2119 g of water were charged into a 3L separable flask, and heated to 80 $^0$C. Then, 73.6 g of 48 mass% sodium hydroxide aqueous solution was added to the flask, and the mixture was stirred at the same temperature (80 $^0$C) for 1 hour to obtain the aqueous solution of the fatty acid alkaline metal salt. 45.8 g of borosilicate glass (Ca/Al) (FTD008FY manufactured by Nippon Sheet Glass Co., Ltd.) having an average particle size of 8.2 $\mu$m was added to the obtained solution, which was then stirred for 1 hour. Thereafter, 178.7 g of 35 mass% zinc chloride aqueous solution was added dropwise to the aqueous solution of the fatty acid alkaline metal salt over a period of 30 minutes while maintaining the temperature at 80 $^0$C. After completing dropwise addition, the mixture was stirred for 30 minutes while maintaining at 80 $^0$C for aging. 1500 g of water was added to the resulted slurry of the fatty acid calcium salt aqueous solution, which was then cooled to 65$^0$C or lower. Thereafter, the slurry was filtered by a suction filer, washed with 1000 g of water 2 times, and the resulted cake was dried with a micron dryer, pulverized and classified to obtain the zinc myristate salt particles having borosilicate glass (Ca/Al) as a nucleating agent.

<Measurement of physical properties of composite particle>

The physical properties of the composite particles obtained in the above Synthesis Examples 1 to 10 were measured by the method described below. The results are shown in Table 1.

{Inside incorporation rate A}

The inside incorporation rate A was obtained by the following Formula as described in the present specification.

$$\text{Inside incorporation rate A} = 100 - (X/X') \times 100$$

$$\text{Formula (1)}$$

[wherein,

X is the surface content of the inorganic nucleating agent in the composite particles,
X' is the whole content of the inorganic nucleating agent in the composite particles,
X and X' are average values obtained by conducting elemental analysis using SEM/EDX on 3 places within the area of 15 $\mu$m square under the condition of the acceleration voltage of 10 kV and the height of 15 mm for the composite particles before and after homogenization respectively]
Homogenization was conducted by pulverization using a mortar.

{D10, D50, and D90}

The D10 (10% cumulative diameter on the basis of the volume), D50 (50% cumulative diameter on the basis of the volume), and D90(90% cumulative diameter on the basis of the volume) were measured using Microtrac MT-3000 manufactured by NIKISO CO., LTD.

{grain size summary value B}

The grain size summary value B was obtained by the following Formula as described in the present specification.

$$\texttt{Grain size summary value B = (D90 - D10)/D50}$$

[Table 1]

| | Component | Inside incorporation rate A(%) | D10 (μm) | D50 (μm) | D90 (μm) | Grain size summary value B |
|---|---|---|---|---|---|---|
| Synthesis Example 1 | Ca myristate-treated -Borosilicate (Ca/Al) | 54.4 | 7.1 | 16.2 | 29.7 | 1.90 |
| Synthesis Example 2 | Ca laurate-treated -Borosilicate (Ca/Al) | 56.9 | 7.1 | 17.5 | 30.3 | 1.79 |
| Synthesis Example 3 | Ca palmitate-treated -Borosilicate (Ca/Al) | 53.3 | 7.5 | 17.9 | 31.4 | 1.80 |
| Synthesis Example 4 | Ca stearate-treated -Borosilicate (Ca/Al) | 57.9 | 7.8 | 18.4 | 33.7 | 1.96 |
| Synthesis Example 5 | Ca myristate-treated -Barium sulfate | 45.5 | 7.1 | 15.3 | 28.6 | 1.87 |
| Synthesis Example 6 | Mg myristate-treated -Borosilicate (Ca/Al) | 32.7 | 38.8 | 96.1 | 171.9 | 1.95 |
| Synthesis Example 7 | Mg myristate-treated -Barium sulfate | 31.2 | 50.5 | 123.8 | 210.9 | 1.73 |
| Synthesis Example 8 | Ca myristate-treated -Talc | 42.7 | 28.3 | 43.5 | 99.7 | 1.98 |
| Synthesis Example 9 | Ca myristate-treated -Boron nitride | 32.8 | 27.6 | 40.4 | 95.3 | 1.99 |
| Synthesis Example 10 | Zn myristate-treated -Borosilicate (Ca/Al) | 43.6 | 10.3 | 21.8 | 41.7 | 1.93 |

<Powder foundation>

The components listed in Table 2-1 and Table 2-2 were mixed uniformly so that the contents (wt%) listed in Table 2-1 and Table 2-2 may be obtained, and subjected to mold filling in an inner tray to provide a powder foundation which is a solid powder cosmetic. Further, the components listed in Table 2-3 were mixed uniformly so that the contents (wt%) listed in Table 2-3 may be obtained, and the slurry was prepared by dispersing the components uniformly in ethanol and filled in an inner tray to provide a powder foundation which is a solid powder cosmetic by wet molding. The functions of the powder foundations were evaluated by the following method. The results are shown in Table 2.

{Formability of powder foundation}

The formability of the powder foundations was compared by installing such cosmetic to the specified compact container after preparing the cosmetic, subjecting the container to front-face dropping from the height of 50 cm onto a plywood having a thickness of 2 cm, and determining the average number of dropping until any of fracture, chipping or crack was generated (n=3).

[Evaluation criteria]

A: 4 times or more
B: not less than 3 times and less than 4 times
C: not less than 2.0 times and less than 3.0 times
D: unable to be formed, or less than 2 times

{Anti-caking of powder foundation}

Caking of the powder foundations was evaluated by rubbing off the surface of such cosmetic by 40 reciprocations using the specified puff after preparing the cosmetic, graded on 4 grades by a panel of 7 experts and compared for the average grading; 4 points: no caking, 3 points: a slight caking but able to be rubbed off without difficulties, 2 points: caking occurs, difficult to be rubbed off with a puff, problems in usability exist, 1 point: caking occurs, impossible to be rubbed off.

[Evaluation criteria]

A: not less than 3.5 to 4.0
B: not less than 2.5 and less than 3.5
C: not less than 1.5 and less than 2.5
D: not less than 1.0 and less than 1.5

{Rubbing off property of powder foundation onto puff}

Rubbing off property of the powder foundations onto a puff was evaluated by a panel of 10 experts using the specified puff after preparing such cosmetic, graded on 4 grades and compared for the average grading; 4 points: very good rubbing off property, 3 points: good rubbing off property, 2 points: slightly poor rubbing off property, 1 point: poor rubbing off property.

[Evaluation criteria]

A: not less than 3.5 to 4.0

B: not less than 2.5 and less than 3.5

C: not less than 1.5 and less than 2.5

D: not less than 1.0 and less than 1.5

{Spreading of powder foundation when applied}

Spreading of the powder foundations when applied was evaluated by a panel of 7 experts after preparing such cosmetic, graded on 4 grades and compared for the average grading; 4 points: very good spreading, 3 points: good spreading, 2 points: slightly poor spreading, 1 point: poor spreading.

[Evaluation criteria]

A: not less than 3.5 to 4.0
B: not less than 2.5 and less than 3.5
C: not less than 1.5 and less than 2.5
D: not less than 1.0 and less than 1.5

{Adherence of powder foundation when applied}

Adherence of the powder foundations when applied was evaluated by a panel of 7 experts after preparing such cosmetic, graded on 4 grades and compared for the average grading; 4 points: very good adherence, 3 points: good adherence, 2 points: slightly poor adherence, 1 point: poor adherence.

[Evaluation criteria]

A: not less than 3.5 to 4.0

B: not less than 2.5 and less than 3.5

C: not less than 1.5 and less than 2.5

D: not less than 1.0 and less than 1.5

{Finish of powder foundation after application}

Finish of the powder foundations after application was evaluated by a panel of 7 experts after preparing such cosmetic, graded on 4 grades and compared for the average grading; 4 points: very good finish, 3 points: good finish, 2 points: slightly poor finish, 1 point: poor finish.

[Evaluation criteria]

A: not less than 3.5 to 4.0

B: not less than 2.5 and less than 3.5

C: not less than 1.5 and less than 2.5

D: not less than 1.0 and less than 1.5

[Table 2-1]

**Powder foundation-1**

| Component | Product name etc. | Supplier | Example | | | | | | | | | Comp arative Example | | | | |
|---|---|---|---|---|---|---|---|---|---|---|---|---|---|---|---|---|
| | | | 1 | 2 | 3 | 4 | 5 | 6 | 7 | 8 | 9 | 1 | 2 | 3 | 4 | 5 |
| Ca myristate-treated -Borosilicate (Ca/Al) | Synthesis Example 1 | | 20 | 30 | | | | | | | 50 | | | | | |
| Ca laurate-treated -Borosilicate (Ca/Al) | Synthesis Example 2 | | | | 30 | | | | | | | | | | | |
| Ca palmitate-treated -borosilicate (Ca/Al) | Synthesis Example 3 | | | | | 30 | | | | | | | | | | |
| Ca stearate-treated -Borosilicate (Ca/Al) | Synthesis Example 4 | | | | | | 30 | | | | | | | | | |
| Ca myristate-treated -Barium sulfate | Synthesis Example 5 | | | | | | | 30 | | | | | | | | |
| Mg myristate-treated -Borosilicate (Ca/Al) | Synthesis Example 6 | | | | | | | | 30 | | | | | | | |
| Mg myristate-treated -Barium sulfate | Synthesis Example 7 | | | | | | | | | 30 | | | | | | |
| Ca myristate-treated -Talk | Synthesis Example 8 | | | | | | | | | | | 30 | | | | |
| Ca myristate-treated -Boron nitride | Synthesis Example 9 | | | | | | | | | | | | 30 | | | |
| Zn myristate-treated -Borosilicate (Ca/Al) | Synthesis Example 10 | | | | | | | | | | | | | 30 | | |

**Powder foundation-1**

| Component | Product name etc. | Supplier | Example | | | | | | | | | Comp arative Example | | | | |
|---|---|---|---|---|---|---|---|---|---|---|---|---|---|---|---|---|
| | | | 1 | 2 | 3 | 4 | 5 | 6 | 7 | 8 | 9 | 1 | 2 | 3 | 4 | 5 |
| Barium sulfate | Barium sulfa-teH | SAKAI CHE-MICAL IN-DUSTRY CO., LTD. | | | | | | | | | | | | | 30 | |
| borosilicate (CalAl) | FTD008Y | Nippon Sheet Glass Co., Ltd. | | | | | | | | | | | | | | 30 |
| Dimethicone-treated Mica | Mica SSA | K. S. PEARL | 52.2 | 42.2 | 42.2 | 42.2 | 42.2 | 42.2 | 42.2 | 42.2 | 22.2 | 42.2 | 42.2 | 42.2 | 42.2 | 42. 2 |
| Hydrogen di-methicone-treated Tita-nium oxide | Titanium oxide DN-SH(2) | Dainihonkasei Co., Ltd. | 9 | 9 | 9 | 9 | 9 | 9 | 9 | 9 | 9 | 9 | 9 | 9 | 9 | 9 |
| spherical silica | SUNSPHERE H-53 | AGC Si-Tech Co., Ltd. | 4.5 | 4.5 | 4.5 | 4.5 | 4.5 | 4.5 | 4.5 | 4.5 | 4.5 | 4.5 | 4.5 | 4.5 | 4.5 | 4.5 |
| spherical silica | SUNSPHERE H-121 | AGC Si-Tech Co., Ltd. | 2 | 2 | 2 | 2 | 2 | 2 | 2 | 2 | 2 | 2 | 2 | 2 | 2 | 2 |
| Dimethicone-treated Yellow iron oxide | YellowSSA | K. S. PEARL | 1.8 | 1.8 | 1.8 | 1.8 | 1.8 | 1.8 | 1.8 | 1.8 | 1.8 | 1.8 | 1.8 | 1.8 | 1.8 | 1.8 |
| Dimethicone-treated colcothar | Red SSA | K. S. PEARL | 0.36 | 0.36 | 0.36 | 0.36 | 0.36 | 0.36 | 0.36 | 0.36 | 0.36 | 0.36 | 0.36 | 0.36 | 0.36 | 0.3 6 |
| Dimethicone-treated Black iron oxide | Black SSA | K. S. PEARL | 0.14 | 0.14 | 0.14 | 0.14 | 0.14 | 0.14 | 0.14 | 0.14 | 0.14 | 0.14 | 0.14 | 0.14 | 0.14 | 0.1 4 |
| Liquid oil (Dimethicone) | KF-96A-20CS | Shin-Etsu Chemical Co., Ltd. | 4.9 | 4.9 | 4.9 | 4.9 | 4.9 | 4.9 | 4.9 | 4.9 | 4.9 | 4.9 | 4.9 | 4.9 | 4.9 | 4.9 |

(continued)

| Powder foundation-1 | | | | | | | | | | | | | | | | | |
|---|---|---|---|---|---|---|---|---|---|---|---|---|---|---|---|---|---|
| Component | Product name etc. | Supplier | Example | | | | | | | | | Comp arative Example | | | | | |
| | | | 1 | 2 | 3 | 4 | 5 | 6 | 7 | 8 | 9 | 1 | 2 | 3 | 4 | 5 |
| Liquid oil (Neopentyl glycol diethylhexanoate) | COSMOL 525 | The Nisshin-Oillio Group, Ltd. | 2 | 2 | 2 | 2 | 2 | 2 | 2 | 2 | 2 | 2 | 2 | 2 | 2 | 2 |
| Liquid oil (Squalane) | Phytosqualane | SOPHIM | 3 | 3 | 3 | 3 | 3 | 3 | 3 | 3 | 3 | 3 | 3 | 3 | 3 | 3 |
| Liquid oil (Di-pentaerythrityl Hexahydrox-ystearate/-Hexastea rate/Hexarosi-nate) | COSMOL 168ARV | The Nisshin-Oillio Group, Ltd. | 0.1 | 0.1 | 0.1 | 0.1 | 0.1 | 0.1 | 0.1 | 0.1 | 0.1 | 0.1 | 0.1 | 0.1 | 0.1 | 0.1 |
| Total | | | 100 | 100 | 100 | 100 | 100 | 100 | 100 | 100 | 100 | 100 | 100 | 100 | 100 | 100 |
| Formability | | | B | A | A | A | A | B | A | B | A | A | C | B | D | D |
| Anti-caking | | | A | A | A | A | B | A | A | A | A | A | A | A | A | A |
| Rubbing-off onto puff | | | A | A | A | A | B | A | A | A | A | C | A | A | A | A |
| Spreading when applied | | | A | A | A | A | A | B | A | A | B | C | B | D | A | B |
| Adherence when applied | | | A | A | B | A | A | B | B | B | A | C | C | D | D | C |
| Finish after application | | | A | A | A | A | A | A | A | A | A | C | B | D | D | C |

EP 4 506 037 A1

[Table 2-2]

| Powder foundation-2 | | | | | | |
|---|---|---|---|---|---|---|
| Component | Product Name etc. | Supplier | Example 10 | Example 11 | Comparative Example 6 | Comparative Example7 |
| Ca myristate-treated-borosilicate (Ca/Al) | Synthesis Example 1 | | 10 | | | |
| Ca myristate-treated-Barium sulfate | Synthesis Example 5 | | | 10 | | |
| Ca myristate-treated -Boron nitride | Synthesis Example 9 | | | | 10 | |
| borosilicate (Ca/Al) | FTD008Y | Nippon Sheet Glass Co., Ltd. | | | | 10 |
| Mica | Mica SSA | K. S. PEARL | 12.4 | 12.4 | 12.4 | 12.4 |
| Sericite | Sericite SSA | K. S. PEARL | 50 | 50 | 50 | 50 |
| Titanium oxide | Titanium oxideDN-SH(2) | Dainihonkasei Co., Ltd. | 9 | 9 | 9 | 9 |
| Silica | SUNSPHERE H-121 | AGC Si-Tech Co., Ltd. | 4.5 | 4.5 | 4.5 | 4.5 |
| Silica | SUNSPHERE H-52 | AGC Si-Tech Co., Ltd. | 1.8 | 1.8 | 1.8 | 1.8 |
| Dimethicone-treated Yellow iron oxide | Yellow SSA | K. S. PEARL | 1.8 | 1.8 | 1.8 | 1.8 |
| Dimethicone-treated colcothar | Red SSA | K. S. PEARL | 0.36 | 0.36 | 0.36 | 0.36 |
| Dimethicone-treated Black iron oxide | Black SSA | K. S. PEARL | 0.14 | 0.14 | 0.14 | 0.14 |
| Liquid oil (Dimethicone) | KF-96A-20CS | Shin-Etsu Chemical Co., Ltd. | 4.9 | 4.9 | 4.9 | 4.9 |
| Liquid oil (Neopentyl glycol diethylhexanoate) | COSMOL 525 | The Nisshin-Oillio Group, Ltd. | 2 | 2 | 2 | 2 |
| Liquid oil (Squalane) | Phytosqualane | SOPHIM | 3 | 3 | 3 | 3 |
| Liquid oil (Dipentaerythrityl Hexahydroxystearate/Hexas tearate/Hexarosinate) | COSMOL 168ARV | The Nisshin-Oillio Group, Ltd. | 0.1 | 0.1 | 0.1 | 0.1 |
| Total | | | 100 | 100 | 100 | 100 |
| Formability | | | B | B | D | C |
| Anti-caking | | | A | A | A | A |
| Rubbing-off onto puff | | | A | A | A | B |
| Spreading when applied | | | B | A | C | B |
| Adherence when applied | | | A | A | C | D |
| Finish after application | | | A | A | C | C |

[Table 2-3]

| Component | Product Name etc. | Supplier | Example 12 |
|---|---|---|---|
| Ca myristate-treated-borosilicate (Ca/Al) | Synthesis Example 1 | | 47.75 |
| Titanium oxide | ST-705SA | Titan Kogyo, Ltd. | 10 |
| Triethoxycaprylylsilane-treated Yellow iron oxide | HP Iron Oxide Yellow 3AS | K. S. PEARL | 1.76 |
| Triethoxycaprylylsilane-treated Colcothar | HP Iron Oxide Red 3AS | K. S. PEARL | 0.35 |
| Triethoxycaprylylsilane-treated Black iron oxide | HP Iron Oxide Black 3AS | K. S. PEARL | 0.14 |
| Spherical silicone particle (Diphenyl dimethicone/Vinyl diphenyl dimethicone/silsesquioxane crosspolymer) | KSP-300 | Shin-Etsu Chemical Co., Ltd. | 30 |
| Liquid oil (Dimethicone) | KF-96A-20CS | Shin-Etsu Chemical Co., Ltd. | 10 |
| | Total | | 100 |
| | Formability | | B |
| | Anti-caking | | A |
| | Rubbing-off onto puff | | A |
| | Spreading when applied | | A |
| | Adherence when applied | | A |
| | Finish after application | | A |

<Liquid foundation>

The components listed in Table 3 were mixed uniformly so that the contents (wt%) listed in Table 3 may be obtained to provide a liquid foundation which is a water-in-oil type emulsified cosmetic. Evaluation was made similarly to powder foundations. The results are shown in Table 3.

[Table 3]

| Liquid foundation | | | | | | | | | | | |
|---|---|---|---|---|---|---|---|---|---|---|---|
| Component | Product Name etc. | Supplier | Example | | | | Comparative Example | | | | |
| | | | 13 | 14 | 15 | 16 | 8 | 9 | 10 | 11 | 12 |
| Dimethicone, Dimethicone/(-PEG-10/15) crosspolymer | KSG-210 | Shin-Etsu Chemical Co., Ltd. | 3 | 3 | 3 | 3 | 3 | 3 | 3 | 3 | 3 |
| Dimethicone, Dimethicone/Vinyl dimethicone crosspolymer | KSG-16 | Shin-Etsu Chemical Co., Ltd. | 2 | 2 | 2 | 2 | 2 | 2 | 2 | 2 | 2 |
| PEG-9 polydimethylsiloxyethyl dimethicone | KF-6028 | Shin-Etsu Chemical Co., Ltd. | 2 | 2 | 2 | 2 | 2 | 2 | 2 | 2 | 2 |
| Lauryl PEG-9 polydimethylsiloxyethyl dimethicone | KF-6038 | Shin-Etsu Chemical Co., Ltd. | 0.5 | 0.5 | 0.5 | 0.5 | 0.5 | 0.5 | 0.5 | 0.5 | 0.5 |
| Isododecane | MARUKASOL R | Maruzen Petrochemical Co., Ltd. | 17.5 | 17.5 | 17.5 | 17.5 | 17.5 | 17.5 | 17.5 | 17.5 | 17.5 |
| Isotridecyl Isononanoate | SALACOS 913 | The Nisshin-Oillio Group, Ltd. | 3 | 3 | 3 | 3 | 3 | 3 | 3 | 3 | 3 |
| Hydrogenated polyisobutene | PEARLEAM 6 | NOF CORPORATION | 2 | 2 | 2 | 2 | 2 | 2 | 2 | 2 | 2 |
| Tocopherol | | | 0.1 | 0.1 | 0.1 | 0.1 | 0.1 | 0.1 | 0.1 | 0.1 | 0.1 |
| Ethylhexylglycerin | sensiva SC 50 JP | SEIWA SUPPLY CO., LTD. | 0.2 | 0.2 | 0.2 | 0.2 | 0.2 | 0.2 | 0.2 | 0.2 | 0.2 |
| Polyglyceryl-2 Isostearate | COSMOL 41V | The Nisshin-Oillio Group, Ltd. | 1 | 1 | 1 | 1 | 1 | 1 | 1 | 1 | 1 |
| Quaternium-18 Bentonite | S-BEN WV | HOJUN CO., LTD. | 1.5 | 1.5 | 1.5 | 1.5 | 1.5 | 1.5 | 1.5 | 1.5 | 1.5 |
| Triethoxycaprylylsilane-treated Titanium oxide | TiO2 CR-50 AS | K. S. PEARL | 7 | 7 | 7 | 7 | 7 | 7 | 7 | 7 | 7 |
| Ca myristate-treated-borosilicate (Ca/Al) | Synthesis Example 1 | | 10 | | | | | | | | |
| Ca myristate-treated-Barium sulfate | Synthesis Example 5 | | | 10 | | | | | | | |
| Mg myristate-treated-borosilicate (Ca/Al) | Synthesis Example 6 | | | | 10 | | | | | | |

EP 4 506 037 A1

(continued)

| Liquid foundation | | | | | | | | | | | |
|---|---|---|---|---|---|---|---|---|---|---|---|
| Component | Product Name etc. | Supplier | Example | | | | Comparative Example | | | | |
| | | | 13 | 14 | 15 | 16 | 8 | 9 | 10 | 11 | 12 |
| Mg myristate-treated-Barium sulfate | Synthesis Example 7 | | | | | 10 | | | | | |
| Ca myristate-treated-Talc | Synthesis Example 8 | | | | | | 10 | | | | |
| Ca myristate-treated-Boron nitride | Synthesis Example 9 | | | | | | | 10 | | | |
| Zn myristate-treated-Borosilicate (Ca/Al) | Synthesis Example 10 | | | | | | | | 10 | | |
| Barium sulfate | Barium sulfateH | SAKAI CHEMICAL INDUSTRY CO., LTD. | | | | | | | | 10 | |
| borosilicate (Ca/Al) | FTD008Y | Nippon Sheet Glass Co., Ltd. | | | | | | | | | 10 |
| Triethoxycaprylylsilane-treated Yellow iron oxide | HP I.O.Yellow 3AS | K.S.PEARL | 1.7 | 1.7 | 1.7 | 1.7 | 1.7 | 1.7 | 1.7 | 1.7 | 1.7 |
| Triethoxycaprylylsilane-treated Colcothar | HP I.O.Red 3AS | K. S. PEARL | 0.32 | 0.32 | 0.32 | 0.32 | 0.32 | 0.32 | 0.32 | 0.32 | 0.32 |
| Triethoxycaprylylsilane-treated Black iron oxide | HP I.O.Black 3AS | K. S. PEARL | 0.15 | 0.15 | 0.15 | 0.15 | 0.15 | 0.15 | 0.15 | 0.15 | 0.15 |
| Purified water | | | 40.13 | 40.13 | 40.13 | 40.13 | 40.13 | 40.13 | 40.13 | 40.13 | 40.13 |
| BG | | | 7 | 7 | 7 | 7 | 7 | 7 | 7 | 7 | 7 |
| Methylparaben | | | 0.1 | 0.1 | 0.1 | 0.1 | 0.1 | 0.1 | 0.1 | 0.1 | 0.1 |
| Phenoxyethanol | | | 0.2 | 0.2 | 0.2 | 0.2 | 0.2 | 0.2 | 0.2 | 0.2 | 0.2 |
| Citric acid | | | 0.1 | 0.1 | 0.1 | 0.1 | 0.1 | 0.1 | 0.1 | 0.1 | 0.1 |
| Sodium citrate | | | 0.5 | 0.5 | 0.5 | 0.5 | 0.5 | 0.5 | 0.5 | 0.5 | 0.5 |
| Total | | | 100 | 100 | 100 | 100 | 100 | 100 | 100 | 100 | 100 |
| Spreading when applied | | | A | A | B | A | C | C | C | B | C |
| Adherence when applied | | | A | A | A | B | C | C | D | D | D |

EP 4 506 037 A1

26

(continued)

| Liquid foundation | | | | | | | | | | | |
|---|---|---|---|---|---|---|---|---|---|---|---|
| Component | Product Name etc. | Supplier | Example | | | | Comparative Example | | | | |
| | | | 13 | 14 | 15 | 16 | 8 | 9 | 10 | 11 | 12 |
| Finish when applied | | | A | A | A | A | C | C | C | D | C |

<Eye shadow>

Components of the indicated name listed in Table 4 were mixed uniformly so that the contents (wt%) listed in Table 4 may be obtained, and subjected to mold filling in an inner tray to provide an eye shadow which is a solid powder cosmetic.

[Table 4]

| Eye shadow | | | | |
|---|---|---|---|---|
| Component | Product Name etc. | Supplier | Example 17 | Example 18 |
| Ca myristate-treated -borosilicate (Ca/Al) | Synthesis Example 1 | | 16 | |
| Mg myristate-treated -Barium sulfate | Synthesis Example 7 | | | 16 |
| Dimethicone-treated Mica | Sericite SSA | K. S. PEARL | 53.15 | 53.15 |
| Titanated mica | Flamenco Ultra Silk 2500 | BASF | 7 | 7 |
| Titanated mica | Flamenco Super Pearl 110C | BASF | 5 | 5 |
| Titanated mica | Flamenco Winter Sparkle 130Q | BASF | 8 | 8 |
| Ultramarines | SunCROMA C43-1810 | DIC | 2.5 | 2.5 |
| Red 226 | SunCROMA C37-038 | DIC | 0.35 | 0.35 |
| Dimethicone-treated Mica | KF-96A-1000CS | Shin-Etsu Chemical Co., Ltd. | 4 | 4 |
| Diisostearyl maleate | COSMOL 222 | The Nisshin-Oillio Group, Ltd. | 1.6 | 1.6 |
| Hydrogenated polydecene | NOMCORT HP-30 | The Nisshin-Oillio Group, Ltd. | 2.4 | 2.4 |
| Total | | | 100 | 100 |

The resulted eye shadow exhibited good rubbing off property by the specified tip while having sufficient formability, and also exhibited excellent spreading when applied, excellent adherence and excellent finish.

<Face powder>

Components of the indicated name listed in Table 5 were mixed uniformly so that the contents (wt%) listed in Table 5 may be obtained to provide a face powder.

[Table 5]

| Face powder | | | | |
|---|---|---|---|---|
| Component | Product Name etc. | Supplier | Example 19 | Example 20 |
| Ca myristate-treated -Barium sulfate | Synthesis Example 5 | | 50 | |
| Mg myristate-treated -borosilicate (Ca/Al) | Synthesis Example 6 | | | 50 |
| Dimethicone-treated Mica | Sericite SSA | K. S. PEARL | 34.9 | 34.9 |
| Butyl acrylate/ Glycol Dimethacrylate crosspolymer, Methyl methacrylate/ Glycol Dimethacrylate crosspolymer | Matsumoto Microsphere S-100 | Matsumoto Yushi Seiyaku Co., Ltd | 7 | 7 |

(continued)

| Face powder | | | | |
|---|---|---|---|---|
| Component | Product Name etc. | Supplier | Example 19 | Example 20 |
| Silica | SUNSPHERE H-51 | AGC Si-Tech Co., Ltd. | 4 | 4 |
| Hydrogen dimethicone-treated zinc oxide microparticle | NANOFINE-50LP | SAKAI CHEMICAL INDUSTRY CO., LTD. | 3 | 3 |
| Methylparaben | | | 0.1 | 0.1 |
| Squalane | Phytosqualane | SOPHIM | 1 | 1 |
| Total | | | 100 | 100 |

The resulted face powder exhibited excellent spreading when applied, excellent adherence and finish.

<Mascara>

Components of the indicated name listed in Table 6 were mixed uniformly so that the contents (wt%) listed in Table 6 may be obtained to provide a mascara.

[Table 6]

| Mascara | | | |
|---|---|---|---|
| Component | Product Name etc. | Supplier | Example 21 |
| Ca myristate-treated -borosilicate (Ca/Al) | Synthesis Example 1 | | 5.8 |
| Dimethicone-treated Black iron oxide | Black SSA | K. S. PEARL | 5.8 |
| Hydrogenated polyisobutene | PEARLEAM 18 | NOF CORPORATION | 2 |
| Isododecane | MARUKASOL R | Maruzen Petrochemical Co., Ltd. | 55.65 |
| Trimethylsiloxysilicate, Cyclopentasiloxane | KF-7312J | Shin-Etsu Chemical Co., Ltd. | 10 |
| Dextrin Palmitate | Rheopearl KL2 | Chiba Flour Milling Co., Ltd. | 15 |
| Microcrystalline wax | Hi-Mic-1090 | NIPPON SEIRO CO., LTD | 2 |
| Polyethylene | SANWAX 171-P | Sanyo Chemical Industries, Ltd. | 3 |
| Tocopherol | | | 0.05 |
| Propylparaben | | | 0.2 |
| Silica Dimethyl Silylate | AEROSIL R972 | NIPPON AEROSIL CO., LTD. | 0.5 |
| Total | | | 100 |

The resulted mascara exhibited excellent adherence to eyelashes and excellent volume up effect.

<Lipstick>

Components of the indicated name listed in Table 7 were mixed uniformly so that the contents (wt%) listed in Table 7 may be obtained to provide a lipstick.

[Table 7]

| Lipstick | | | |
|---|---|---|---|
| Component | Product Name etc. | Supplier | Example 22 |
| Carnauba wax | Purified Carnauba wax No.1 | CERARICA NODA Co., Ltd. | 2 |
| Candelilla wax | Purified Candelilla wax No.1 | CERARICA NODA Co., Ltd. | 5 |
| Ceresin | CERESIN #810 | NIKKO RICA CORPORATION | 6 |
| Microcrystalline wax | Multiwax W-445 | Sonneborn | 2 |
| Cholesteryl Hydroxystearate | SALACOS HS | The Nisshin-Oillio Group, Ltd. | 1.5 |
| Dipentaerythrityl Hexahydroxystearate/Hexastearate/Hexarosinate | COSMOL 168ARV | The Nisshin-Oillio Group, Ltd. | 12 |
| Diisostearyl maleate | COSMOL 222 | The Nisshin-Oillio Group, Ltd. | 18 |
| Polyglyceryl-2 triisostearate | COSMOL 43V | The Nisshin-Oillio Group, Ltd. | 8.2 |
| Neopentyl glycol dicaprate | COSMOL N-01 | The Nisshin-Oillio Group, Ltd. | 12 |
| 2-Octyl dodecanol | KALKOL 200GD | Kao Corporation | 15 |
| Tocopherol | | | 0.1 |
| Propylparaben | | | 0.1 |
| Titanium oxide microparticle 48% dispersion | Cosmeserve WP-58MP(V) | Dainihonkasei Co., Ltd. | 3 |
| Ca myristate-treated-borosilicate (Ca/Al) | Synthesis Example 1 | | 2.5 |
| Titanium oxide-coated Mica | Flamenco Sparkle Gold | BASF | 4 |
| Titanium oxide-coated Maica | Flamenco Super Pearl 120C | BASF | 2 |
| Pigment Res 57-1 40% dispersion (Dispersion media: Polyglyceryl-2 Triisostearate) | | | 1.4 |
| colcothar 55% dispersion (Dispersion media: Diisostearyl maleate) | | | 5 |
| Blue 1 45% dispersion (Dispersion media: Diisostearyl maleate) | | | 0.3 |
| Total | | | 100 |

The resulted lipstick exhibited excellent spreading when applied, excellent adherence to lips and excellent finish.

<Skin care milky lotion>

Components of the indicated name listed in Table 8 were mixed uniformly so that the contents (wt%) listed in Table 8 may be obtained to provide a skin care milky lotion.

[Table 8]

| Skin care milky lotion | | | |
|---|---|---|---|
| Component | Product Name etc. | Supplier | Example 23 |
| Water | | | 75.6 |
| Glycerin | | | 3 |
| Polysorbate 20 | RHEODOL TW-L120 | Kao Corporation | 0.5 |
| Phenoxyethanol | | | 0.5 |
| Acrylates/C10-30 alkyl acrylate crosspolymer | Pemulen EZ-4U | Lubrizol Japan Limited | 0.2 |
| PEG-11 methyl ether dimethicone | KF-6011P | Shin-Etsu Chemical Co., Ltd. | 0.1 |
| EDTA-2Na | | | 0.05 |
| Xanthan gum | ECHO GUM | MP Gokyo Food & Chemical Co., Ltd. | 0.05 |
| Triethylhexanoin | T.I.O | The Nisshin-Oillio Group, Ltd. | 4 |
| Di(hytosteryl/octyldodecyl) lauroyl glutamate | ELDEW PS-203 | AJINOMOTO HEALTHY SUPPLY CO., INC. | 0.5 |
| Caprylic/Capric triglyceride | O.D.O | The Nisshin-Oillio Group, Ltd. | 2 |
| Isostearic acid | Isostearic acid EX | KOKYU ALCOHOL KOGYO CO., LTD. | 1 |
| Pentaerythrityl tetraethylhexanoate | SALACOS 5408 | The Nisshin-Oillio Group, Ltd. | 3 |
| Polyacrylamide, C13-14 isoparaffin, Laureth-7 | SEPIGEL 305 | SEIWA KASEI CO., LTD. | 0.5 |
| Sodium hydroxide 5% aqueous solution | | | 1 |
| Propanediol | Zemea Select Propanediol | DuPont Tate & Lyle Bio Products | 3 |
| Pentylene glycol | Hydrolite 5 green | Symrise AG | 2 |
| Ca myristate-treated-borosilicate (Ca/Al) | Synthesis Example 1 | | 3 |
| Total | | | 100 |

The resulted skin care milky lotion exhibited excellent spreading when applied, excellent slip property after application and excellent finish after application.

<Shake well sunscreen>

Components of the indicated name listed in Table 9 were mixed uniformly so that the contents (wt%) listed in Table 9 may be obtained to provide a shake well sunscreen.

[Table 9]

| Shake well sunscreen | | | |
|---|---|---|---|
| Component | Product Name etc. | Supplier | Example 24 |
| Ethylhexyl methoxy cinnamate | Uvinul MC 80 | BASF | 7 |
| Bis-ethylhexyloxyphenol methoxyphenyl triazine | TINOSORB S | BASF | 1 |

(continued)

| Shake well sunscreen | | | |
|---|---|---|---|
| Component | Product Name etc. | Supplier | Example 24 |
| Diethylamino hydroxybenzoyl hexyl benzoate | Uvinul A Plus Granular | BASF | 2 |
| Isopropyl lauroyl sarcosinate | ELDEW SL-205 | AJINOMOTO HEALTHY SUPPLY CO., INC. | 5 |
| Isostearic acid | Isostearic acid EX | KOKYU ALCOHOL KOGYO CO., LTD. | 0.3 |
| Polyhydroxystearic acid | SALACOS HS-6C | The Nisshin-Oillio Group, Ltd. | 0.5 |
| Lauryl PEG-9 polydimethylsiloxyethyl dimethicone | KF-6038 | Shin-Etsu Chemical Co., Ltd. | 1.5 |
| Tocopherol | | | 0.05 |
| Caprylic/Capric Triglyceride | O.D.O | The Nisshin-Oillio Group, Ltd. | 4 |
| Dimethicone/Vinyl dimethicone crosspolymer, Dimethicone | KSG-016F | Shin-Etsu Chemical Co., Ltd. | 3 |
| Titanium oxide microparticle 40% dispersion | Cosmeserve WP-40TK | Dainihonkasei Co., Ltd. | 10 |
| Zinc oxide microparticle 60% dispersion | ZD-300 | Titan Kogyo, Ltd. | 30 |
| Caprylyl Methicone | DOWSIL SS-3408 | Dow Torav Co.,Ltd. | 4 |
| Cyclopentasiloxane | CY-5 | Shin-Etsu Chemical Co., Ltd. | 5.7 |
| Ca myristate-treated-borosilicate (Ca/Al) | Synthesis Exam-ple 1 | | 5 |
| EDTA-2Na | | | 0.05 |
| Na chloride | | | 0.5 |
| Phenoxyethanol | | | 0.4 |
| Water | | | 20 |
| Total | | | 100 |

The resulted shake well sunscreen exhibited excellent spreading when applied, excellent slip property after application and excellent finish after application.

<Sunscreen cream>

Components of the indicated name listed in Table 10 were mixed uniformly so that the contents (wt%) listed in Table 10 may be obtained to provide a sunscreen cream.

[Table 10]

| Sunscreen Cream | | | |
|---|---|---|---|
| Component | Product Name etc. | Supplier | Example 25 |
| Ethylhexyl methoxy cinnamate | Uvinul MC 80 | BASF | 7 |
| Diethylamino hydroxybenzoyl hexyl benzoate | Uvinul A Plus Granular | BASF | 2 |
| Bis-ethylhexyloxyphenol methoxyphenyl triazine | TINOSORBS | BASF | 1 |
| Cyclopentasiloxane | CY-5 | Shin-Etsu Chemical Co., Ltd. | 5.5 |

32

(continued)

| Sunscreen Cream | | | |
|---|---|---|---|
| Component | Product Name etc. | Supplier | Example 25 |
| Neopentyl glycol diethylhexanoate | COSMOL 525 | The Nisshin-Oillio Group, Ltd. | 5 |
| Stearic acid | Purified stearic acid 750V | Kao Corporation | 2.5 |
| Polysorbate 65 | RHEODOL TW-S320V | Kao Corporation | 1 |
| Glyceryl Stearate SE | EMALEX GMS-ASE | NIHON EMULSION Co., Ltd. | 1 |
| Cetearyl Alcohol | KALKOL 6850 | Kao Corporation | 1 |
| Glycol Stearate | EMALEX EGS-A | NIHON EMULSION Co., Ltd. | 2 |
| Tocopherol | | | 0.05 |
| Water | | | 47.63 |
| Glycerin | | | 1 |
| Propanediol | ZEMEA Select Propanediol | DuPont Tate & Lyle Bio Products | 6 |
| Polysorbate 60 | RHEODOL TW-S120V | Kao Corporation | 0.1 |
| Arginine | | | 0.7 |
| Bentonite | BEN-GEL | HOJUN CO., LTD. | 0.9 |
| Xanthan gum | ECHO GUM | MP Gokyo Food & Chemical Co., Ltd. | 0.12 |
| Phenoxyethanol | Phenoxyethanol | | 0.5 |
| Silica-treated Titanium oxide microparticle | ST-455WS | Titan Kogyo, Ltd. | 6 |
| Ca myristate-treated-borosilicate (Ca/Al) | Synthesis Example 1 | | 5 |
| Methylene Bis-Benzotriazolyl Tetramethylbutylphenol aqueous dispersion | TINOSORB M | BASF | 4 |
| Total | | | 100 |

The resulted sunscreen cream exhibited excellent spreading when applied, excellent slip property after application and excellent finish after application.

**Claims**

1. A cosmetic comprising composite particles, wherein

the composite particles contain an inorganic nucleating agent and a metal soap;
the inorganic nucleating agent is at least one selected from the group consisting of glass particles and sparingly soluble sulfate salt particles; and
the metal soap is a salt of a fatty acid and at least one selected from the group consisting of calcium and magnesium.

2. The cosmetic according to claim 1, wherein an amount of the inorganic nucleating agent in the composite particles is 0.1 wt% or more and 30 wt% or less.

3. The cosmetic according to claim 1 or 2, wherein an inside incorporation rate A represented by the following Formula (1) is 25% or more:

$$\texttt{Inside incorporation rate A = 100 - (X/X') × 100}$$

$$\texttt{Formula (1)}$$

wherein,

X is a surface content of the inorganic nucleating agent in the composite particles,
X' is a whole content of the inorganic nucleating agent in the composite particles,
X and X' are average values obtained by conducting elemental analysis using SEM/EDX on 3 places within an area of 15 $\mu$m square under the condition of an acceleration voltage of 10 kV and a height of 15 mm for the composite particles before and after homogenization respectively.

4. The cosmetic according to any one of claims 1 to 3, wherein a grain size summary value B represented by the following Formula (2) is 3 or less:

$$\texttt{grain size summary value B = (D90 - D10)/D50}$$

$$\texttt{Formula (2)}$$

wherein,

D10 is a 10% cumulative diameter ($\mu$m) of the composite particles on the basis of volume,
D50 is a 50% cumulative diameter ($\mu$m) of the composite particles on the basis of volume, and
D90 is a 90% cumulative diameter ($\mu$m) of the composite particles on the basis of volume.

5. The cosmetic according to any one of claims 1 to 4, wherein the glass particles are silicate glass particles.

6. The cosmetic according to any one of claims 1 to 5, wherein the inorganic nucleating agent is at least one selected from the group consisting of borosilicate glass particles and barium sulfate particles.

7. The cosmetic according to any one of claims 1 to 6, wherein the number of carbon atoms of the fatty acid is 8 or more and 22 or less.

8. The cosmetic according to any one of claims 1 to 7, wherein an amount of the composite particles in the cosmetic is 1 wt% or more and 75 wt% or less.

9. The cosmetic according to any one of claims 1 to 8, wherein

the cosmetic further contains an oily component, and
an amount of the oily component in the cosmetic is 0.1 wt% or more and 20 wt% or less.

10. The cosmetic according to any one of claims 1 to 9, wherein

the cosmetic further contains spherical particles,
50% cumulative value of the spherical particles on the basis of volume is 1 $\mu$m or more and 30 $\mu$m or less, and
an amount of the spherical particles in the cosmetic is 1 wt% or more.

11. The cosmetic according to any one of claims 1 to 10, wherein the cosmetic is a solid powder cosmetic.

## INTERNATIONAL SEARCH REPORT

| International application No. |
|---|
| **PCT/JP2023/012586** |

### A. CLASSIFICATION OF SUBJECT MATTER

*A61Q 1/06*(2006.01)i; *A61Q 1/10*(2006.01)i; *A61Q 1/12*(2006.01)i; *A61Q 17/04*(2006.01)i; *A61Q 19/00*(2006.01)i; *A61K 8/23*(2006.01)i; *A61K 8/25*(2006.01)i; *A61K 8/36*(2006.01)i
FI:   A61K8/25; A61Q1/10; A61Q1/12; A61Q1/06; A61Q19/00; A61Q17/04; A61K8/23; A61K8/36

According to International Patent Classification (IPC) or to both national classification and IPC

### B. FIELDS SEARCHED

Minimum documentation searched (classification system followed by classification symbols)

A61Q1/06; A61Q1/10; A61Q1/12; A61Q17/04; A61Q19/00; A61K8/23; A61K8/25; A61K8/36

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

Mintel GNPD

### C. DOCUMENTS CONSIDERED TO BE RELEVANT

| Category* | Citation of document, with indication, where appropriate, of the relevant passages | Relevant to claim No. |
|---|---|---|
| X | WO 2019/189796 A1 (SHISEIDO CO LTD) 03 October 2019 (2019-10-03) claims, paragraphs [0010], [0014]-[0016], [0025]-[0029], [0048], [0054], tables 1, 4, examples 1-6, 10-12 | 1-2, 4, 6-8, 10 |
| A | | 3, 5, 9, 11 |
| X | JP 2017-186323 A (KOSE CORP) 12 October 2017 (2017-10-12) claims, paragraphs [0067]-[0068], example 11 | 1-4, 6-11 |
| A | | 5 |
| A | JP 2011-207880 A (LVMH RECHERCHE) 20 October 2011 (2011-10-20) entire text | 1-11 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 May 2023** | **13 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/012586**

| Patent document cited in search report | | | Publication date (day/month/year) | Patent family member(s) | | Publication date (day/month/year) |
|---|---|---|---|---|---|---|
| WO | 2019/189796 | A1 | 03 October 2019 | US 2021/0093544 A1 claims, paragraphs [0011], [0017]-[0020], [0029]-[0034], [0063], [0066], tables 1, 4, examples 1-6, 10-12 CN 111902121 A | | |
| JP | 2017-186323 | A | 12 October 2017 | (Family: none) | | |
| JP | 2011-207880 | A | 20 October 2011 | US 2011/0236331 A1 entire text DE 102011001611 A1 KR 10-2011-0109899 A | | |

Form PCT/ISA/210 (patent family annex) (January 2015)

36

**REFERENCES CITED IN THE DESCRIPTION**

**Patent documents cited in the description**

- WO 2016132967 A